Europäisches Patentamt

(19) European Patent Office

Office européen des brevets

(11) **EP 0 395 471 B2**

(12) **NOUVEAU FASCICULE DE BREVET EUROPEEN**

(45) Date de publication et mention de la décision concernant l'opposition:
**28.08.1996 Bulletin 1996/35**

(45) Mention de la délivrance du brevet:
**18.08.1993 Bulletin 1993/33**

(21) Numéro de dépôt: **90401017.0**

(22) Date de dépôt: **12.04.1990**

(51) Int. Cl.[6]: **B01J 23/00**, B01J 23/80, C01B 3/22, C07C 29/15, C01G 9/00

(54) **Procédé de préparation de précurseurs de catalyseurs contenant du cuivre, de l'aluminium et du zinc, utilisables pour la synthèse et la décomposition du méthanol**

Verfahren zur Herstellung von Kupfer, Aluminium und Zink enthaltenden Katalysator-Vorläufern für die Synthese und die Spaltung von Methanol

Process for the preparation of catalyst precursors containing copper, aluminium and zinc, useful for synthesis and decomposition of methanol

(84) Etats contractants désignés:
**BE DE FR GB IT NL SE**

(30) Priorité: **24.04.1989 FR 8905424**

(43) Date de publication de la demande:
**31.10.1990 Bulletin 1990/44**

(73) Titulaire: **INSTITUT FRANCAIS DU PETROLE F-92502 Rueil-Malmaison (FR)**

(72) Inventeurs:
• **Chaumette, Patrick**
  **F-78380 Bougival (FR)**
• **Le Peltier, Fabienne**
  **F-92500 Rueil Malmaison (FR)**
• **Szymanski, Raymond**
  **F-78600 Maisons Laffitte (FR)**
• **Courty, Philippe**
  **F-78800 Houilles (FR)**

• **Verdon, Catherine**
  **F-92500 Rueil-Malmaison (FR)**

(74) Mandataire: **Lewald, Dietrich, Dipl.-Ing. et al Lewald.Grape.Schwarzensteiner Patentanwälte Rindermarkt 6 D-80331 München (DE)**

(56) Documents cités:
EP-A- 0 109 703    EP-A- 0 146 165
EP-A- 0 152 314    DE-A- 3 717 111
GB-A- 1 405 012    US-A- 3 546 140
US-A- 4 279 781    US-A- 4 386 059

• STUDIES IN SURFACE SCIENCE AND CATLYSIS, vol. 31, 1987, pages 767-783, Elsevier Science Publishers B.V., Amsterdam, NL; E.B.M. DOESBURG et al.: "Preparation and characterization of copper/zinc oxide/alumina catalysts for methanol synthesis"

## Description

La présente invention concerne un procédé de préparation d'un précurseur, de préférence hydroxycarbonaté, d'un catalyseur utilisable notamment dans des procédés mettant en jeu une réaction équilibrée des oxydes de carbone (CO, $CO_2$) avec l'hydrogène, en particulier la production de méthanol à partir de gaz de synthèse et les réactions de décomposition des alcools primaires et en particulier du méthanol ou de l'éthanol en un mélange contenant des oxydes de carbone et de l'hydrogène.

Ce précurseur est formé selon l'invention par l'association ou le mélange par exemple à l'échelle du micromêtre ou à une échelle inférieure au micromètre de deux précurseurs ternaires (soit sous forme hydraté, soit sous forme oxyde si ils ont subi une calcination finale) comprenant chacun les trois métaux suivants : le cuivre, l'aluminium et le zinc. L'association (ou mélange) de ces deux précurseurs ternaires est notamment caractérisée par le fait que lesdits précurseurs ternaires diffèrent l'un de l'aube à la fois par leur stoéchiométrie métallique (rapports atomiques entre Cu,Zn et Al), par leur structure cristallographique et par leur morphologie élémentaire. Ce mélange desdits précurseurs ternaires peut se faire à différents stades de leur fabrication.

Les catalyseurs à base d'oxydes de cuivre et de zinc sont connus depuis de nombreuses années; ils ont été décrits dès 1933 par DODGE (US-A-1 908 696). Dans les brevets US-A-3 388 972, 3 546 140 et 3 790 505, la société américaine C.C.I. décrit l'utilisation de compositions ternaires Cu, Zn, Al pour la conversion à basse température du monoxyde de carbone (CO) et pour la synthèse du méthanol.

Divers modes de préparation des catalyseurs Cu, Zn, Al sont décrits notamment dans US 3 923 694, 4 279 781, 4 596 782 et FR-A-2 352 588 .

FR-A-2 352 588 décrit la préparation d'un catalyseur par mélange mécanique de 10 à 60% d'oxyde de cuivre, 5 à 40% d'oxyde de zinc et 30 à 70% d'un ciment alumineux, ainsi que l'utilisation de ce catalyseur pour la conversion du monoxyde de carbone afin de produire de l'hydrogène ou du méthanol.

Exception faite du catalyseur décrit dans FR-A-2 352 588, ces catalyseurs sont généralement produits par réaction de précipitation entre une solution acide contenant par exemple les nitrates des métaux précités et une solution basique contenant par exemple un carbonate alcalin. La réaction de précipitation, tel que décrite par exemple dans le brevet américain US-A-3 923 694, conduit à l'obtention de précurseurs hydratés, cristallisés au moins pour partie, qui sont constitués d'au moins trois phases : une phase ternaire CuAlZn, avec $\frac{Cu + Zn}{Al}$ = 3 (atomes.atome$^{-1}$), de type hydrotalcite; une phase binaire, la rosacite, qui est un hydroxycarbonate mixte de cuivre et de zinc; et un hydroxycarbonate de cuivre, la malachite, ainsi qu'éventuellement d'autres phases, comme par exemple le spinelle $ZnAl_2O_4$ décrit dans le brevet US-A-3 923 694. L'hétérogénéité de composition de ces catalyseurs a pour conséquence une activité, une sélectivité et une stabilité relativement faibles, même si, initialement, ils contiennent un oxyde de cuivre bien dispersé, au moins en partie. On trouve dans diverses publications, par exemple dans F. TRIFIRO et al, Preparation of Catalysts III, p.723-733, 1983, Elsevier Science Publishers (Amsterdam), une description détaillée de la formation simultanée de ces phases.

La préparation de catalyseurs à base de cuivre obtenus par calcination de précurseurs cristallisés identifiés, et plus particulièrement de phases hydroxycarbonates obtenues par coprécipitation, a été décrite dans de rares cas. Le brevet US-A-4 145 400 décrit la préparation de catalyseurs CUZnAl, mais lesdits catalyseurs sont préparés à partir d'un seul précurseur monophasique cristallisé, l'hydrotalcite. Le brevet US-A-4 596 782 décrit quant à lui la préparation d'un catalyseur de synthèse du méthanol à partir d'un seul précurseur hydraté amorphe d'une très grande homogénéité. Le catalyseur décrit dans le brevet US-A-4 436 833 est d'autre part préparé à partir d'un mélange d'une phase cristallisée binaire de formulation $Cu_{2,2}Zn_{2,8}(OH)_{16}(CO_3)_2$ et d'hydroxyde d'aluminium. Enfin, le brevet US-A-3 923 694 décrit une coprécipitation séquentielle où un précurseur de spinelle contenant de l'aluminium et du zinc est d'abord obtenu, puis où on coprécipite sur ce précurseur un composé Cu-Zn binaire. Dans de nombreux documents, par exemple US-A-3 388 972, 3 546 140, FR-A-2 027 162, l'alumine, composant essentiel du catalyseur, est incorporée à l'état d'oxyde ou encore, à l'état d'hydroxyde (US-A-4 279 781).

Le brevet anglais GB-A-1405012 décrit une méthode de fabrication d'une composition transformable en un précurseur de catalyseur.

Cette composition est obtenue en formant un premier précipité fait à partir d'oxydes de zinc et d'aluminium et jusqu'à 10% atomique d'oxyde de cuivre formant ensemble une structure spinelle et un deuxième précipité fait à partir d'un composé de cuivre, de préférence accompagné d'un composé de zinc et eventuellement d'aluminium.

Le précurseur, de préférence hydroxycarbonaté, peut être obtenu selon l'invention par mélange de deux précurseurs ternaires, de préférence hydroxycarbonatés, comprenant chacun au moins du cuivre, de l'aluminium et du zinc, un desdits précurseurs ternaires contenant au moins 50%, de préférence au moins 65%, et de manière encore plus préférée, au moins 85% en poids d'une phase appelée "rodérite" définie ci-après, et l'autre desdits précurseurs ternaires contenant au moins 50%, de préférence au moins 65% et, de manière encore plus préférée, au moins 85% en poids d'une phase appelée "prespinelle" définie ci-après.

La phase rodérite a apparemment déjà été observée, mais mal caractérisée par DOESBURG E.B.M. (Univ. DELFT), HOPPENER (DSM, Studies in Surface Science and Catalysis Vol. 31, p.767, 1987), toutefois les mêmes

2

auteurs indiquent que les catalyseurs les plus actifs ne contiennent pas cette phase, mais un mélange des phases rosacite et hydrotalcite. L'une de ces deux phases (rosacite) n'est d'ailleurs constituée que des deux éléments cuivre et zinc, contrairement aux deux précurseurs ternaires employés dans la présente invention qui contiennent les trois éléments Cu,Zn,Al.

Comme le montrent les microanalyses par spectrométrie d'émission X menées par la demanderesse, les phases élémentaires rodérite et prespinelle microcristallisée présentent une composition ternaire différente en Cu, Zn et Al :

- la phase rodérite présente un rapport atomique $\frac{Cu+Zn}{Al}$ compris entre 3,5 et 16 et, de préférence, entre 4 et 13 et un rapport atomique Zn/Al compris entre 1,6 et 6 et, de préférence, entre 2 et 5;
- la phase prespinelle microcristallisée présente un rapport atomique $\frac{Cu+Zn}{Al}$ compris entre 0,20 et 2,10 et, de préférence, entre 0,25 et 1,50 et un rapport atomique Zn/Al compris entre 0,10 et 1,50 et, de préférence, entre 0,15 et 1,20.

Lesdites phases élémentaires, les précurseurs et les catalyseurs de la présente invention peuvent être caractérisés par spectrométrie d'émission X dans un microscope électronique à balayage par transmission (MEBT). Les analyses sont réalisées par exemple à l'aide d'un appareil capable de donner des images hautes résolution (0,5 à 1 nm) dans le mode balayage (mode STEM dans la terminologie anglo-saxonne) et possédant une sensibilité élevée dans le mode microanalyse par rayon X. Un appareil commercial tel que le MEBT Vacuum Generator HB 501 équipé d'un détecteur Si-Li KEVEX associé à un analyseur TRACOR convient tout à fait (sensibilité limite meilleure que 1000 atomes d'un élément donné) pour déterminer la morphologie et la composition locale des précurseurs et catalyseurs. Afin de préserver l'agencement des phases entre elles et de mieux décrire leur répartition macroscopique dans les grains de coprécipité lors de la préparation des précurseurs (voir ci-après), on peut utiliser une méthode de préparation par coupe à l'ultramicrotome pour obtenir des sections de grain de quelques dizaines de nanomêtres d'épaisseur (1 nm = $10^{-9}$ m).

Ainsi, après sélection de la zone à analyser (typiquement 2 à 5 nm), on effectue simultanément plusieurs comptages, de durée 100-1000 s, conduisant à une statistique de comptage suffisamment précise (meilleure que 10%).

A partir des intensités mesurées sur les divers pics sélectionnés pour les divers éléments présents dans l'échantillon, on peut déterminer leurs concentrations relatives, puis leurs rapports atomiques respectifs, selon des techniques bien connues en émission X (voir par exemple REED S.J.B. Electron microprobe Analysis, Cambridge University Press, 1975), pour chacune des particules constituant l'échantillon.

Les échantillons comparés doivent tous présenter la même épaisseur. Les valeurs moyennes des coefficients de correction (ramenés à Cu-K$\alpha$ = 1) sont les suivantes :

| Mesures sur la raie | Elément | Coefficient |
| --- | --- | --- |
| K$\alpha$ | cuivre | 1 |
| K$\alpha$ | aluminium | 4,86 |
| K$\beta$ | zinc | 4,68 |

Ces coefficients ont été déterminés par le demandeur à partir d'oxydes mixtes calcinés à haute température : $CuAl_2O_4$, $ZnAl_2O_4$, $Cu_{1-x}Zn_xAl_2O_4$ (x=0,25-0,50-0,75) constituent les échantillons de référence.

Le rapport atomique Zn/Al est calculé par exemple comme suit ($iK_\beta Zn$ et $iK_\alpha Al$ sont les intensités brutes moyennes sur plusieurs comptages) :

$$Zn/Al = 0{,}963 \; i_{K_\beta} Zn / i_{K_\alpha} Al$$

Les meilleurs résultats, en terme d'activité, de sélectivité et de stabilité, sont généralement obtenus avec des catalyseurs pour lesquels chaque monophase (rodérite, prespinelle) présente une variation des rapports atomiques Cu+Zr/Al et Zn/Al inferieure à 15% environ et préférentiellement inférieure à 10% environ par rapport à la valeur moyenne de ce rapport, à l'échelle de 5 nanomètres.

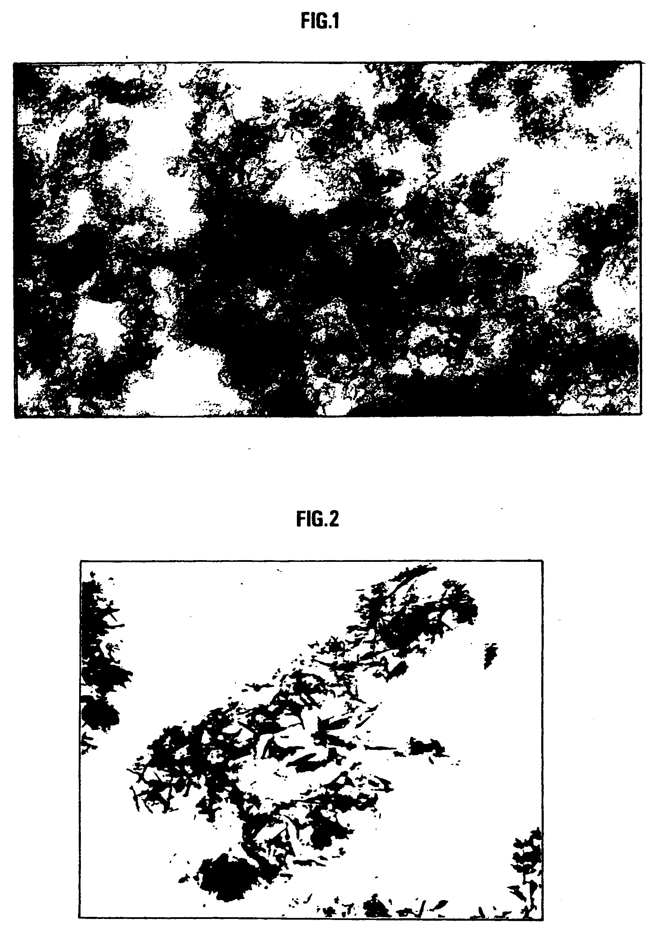

La phase prespinelle microcristallisée se présente sous un aspect granité peu contrasté (figure 1, où 1 cm représente 100 nm) alors que la phase rodérite présente une morphologie fibrillaire, les fibres ayant une longueur comprise entre environ 50 et $900.10^{-10}$m et un diamètre compris entre environ 5 et $100.10^{-10}$m (figure 2, où 1 cm représente 48 nm). Il est donc possible de distinguer ces deux phases par microscopie électronique et de déterminer leurs rapports atomiques $\frac{Cu+Zn}{Al}$ et Zn/Al. Chacune des phases présente une composition homogène au moins à l'échelle du nanomètre, contrôlée par spectrométrie d'émission X.

La diffraction des rayons X peut également être mise en oeuvre pour caractériser les phases rodérite et prespinelle, les précurseurs et les catalyseurs. Les diagrammes de diffraction des rayons X de chaque échantillon sont par exemple enregistrés sur un goniomètre Philips PW 1050 utilisant la raie K$\alpha$ du cobalt L'étude des diagrammes de diffraction des rayons X met en évidence certaines caractéristiques des 2 phases prespinelle et rodérite, de préférence hydroxycarbonatées :

- la phase prespinelle, micro-cristallisée est identifiée à l'état séché par un fond amorphe important et des bandes larges situées à 4,7; 2,87; 2,49 et $1,43.10^{-10}$m;
- la phase rodérite est aisément identifiée à l'état séché à partir des raies situées à 6,8; 4,5 et $3,3.10^{-1o}$m ; ces deux dernières raies permettent notament de la distinguer des structures hydroxycarbonatées de zinc $Zn_5(OH)_6(CO_3)_2$ et $Zn_4(OH)_2(CO_3)_3 4H_2O$.

Une autre technique permettant de confirmer la présence des deux phases prespinelle et rodérite, de préférence hydroxycarbonatées dans un échantillon coprécipité ou séché consiste en l'enregistrement d'un spectre infra-rouge dudit échantillon (avant calcination). Les spectres infra-rouge sont réalisés à l'aide d'un spectromètre, par exemple du type DIGILAB FTS 15E, permettant une analyse par transformée de Fourrier du signal (F.T.-I.R., Fourrier Transformed-Infra Red spectroscopy), l'échantillon (1 mg) étant mélangé avec KBr (300 mg) puis pastillé :

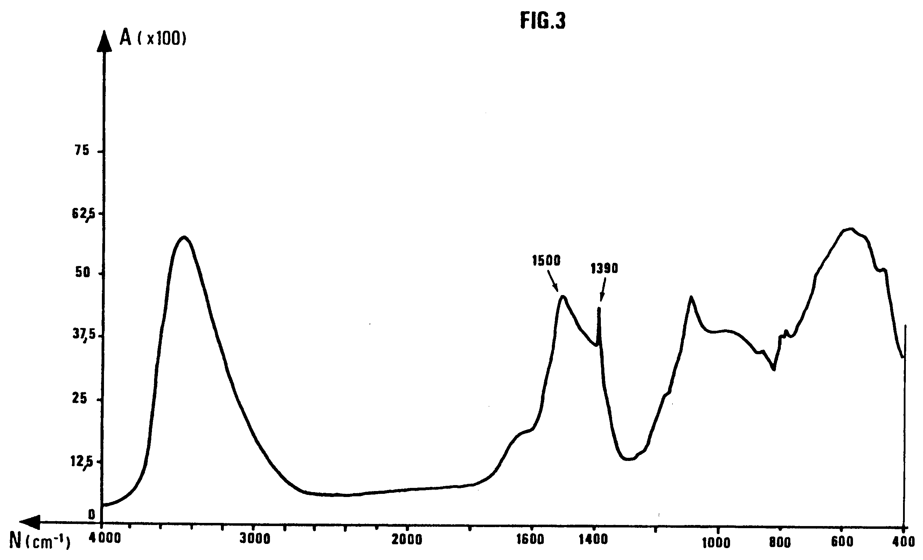

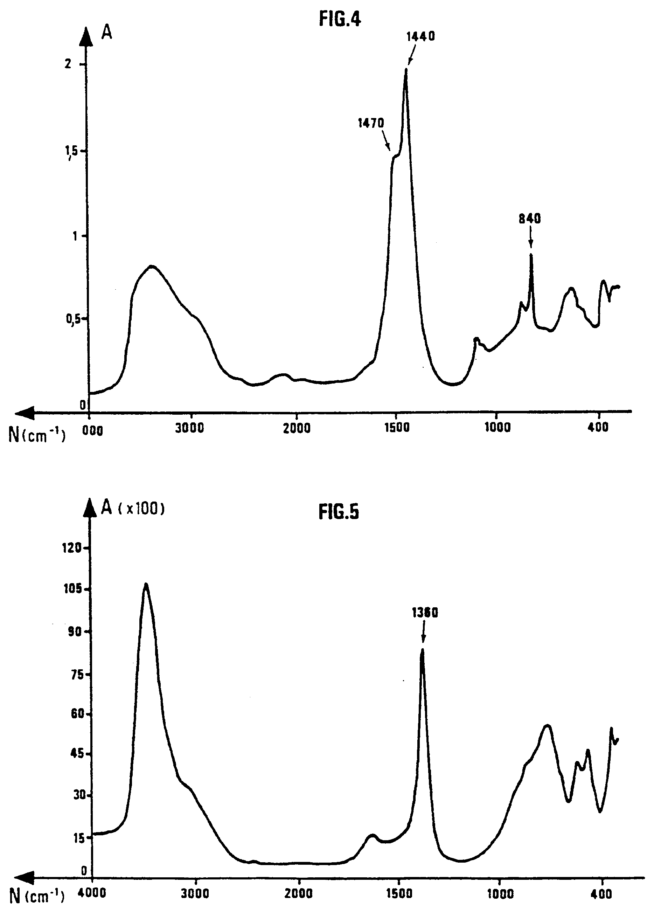

- la phase prespinelle est caractérisée par une vibration $\nu(CO_3^{2-})$, conduisant à une bande large dont le maximum est situé à 1500 cm$^{-1}$ et une bande fine à 1390 cm$^{-1}$ (tableau I et figure 3);
- la phase rodérite présente en revanche des vibrations $\nu(CO_3^{2-})$ sous forme d'un doublet à 1440 et 1470 cm$^{-1}$ et d'un pic fin à 840 cm$^{-1}$ (tableau I et figure 4).

Ces bandes permettent donc de détecter la présence de ces deux phases et de les distinguer de la phase hydrotalcite décrite notamment dans le brevet US-A-4 145 400 dont la vibration caractéristique des groupements carbonates, $\nu(CO_3^{2-})$, est située à 1360 cm$^{-1}$ (tableau I et figure 5). Sur les figures 3 à 5, A représente l'absorbance et N le nombre d'ondes (en cm$^{-1}$).

TABLEAU I

| PHASE | | $\nu(CO_3^{2-})$ | (cm$^{-1}$) |
|---|---|---|---|
| PRESPINELLE | | 1390 | 1500 |
| RODERITE | 840 | 1440 | 1470 |
| HYDROTALCITE | - | 1360 | - |

La phase rodérite et la phase prespinelle microcristallisée représentent ensemble au moins 50%, de préférence au moins 65%, et de manière encore plus préférée, au moins 85% en poids du précurseur, de préférence hydroxycarbonaté , préparé selon l'invention. Lorsque l'association intime rodérite-prespinelle ne constitue pas 100% en poids dudit précurseur, le complément à 100% pourra être formé par :

- une ou plusieurs phases amorphes ou microcristallisés, non détectables par diffraction des rayons X, comprenant au moins un des métaux choisis dans le groupe constitué par le cuivre, l'aluminium et le zinc, et/ou
- une ou plusieurs phases cristallisées telles que l'hydrotalcite, la rosacite, la malachite, l'hydrozincite (la présence d'hydroxynitrate basique de cuivre (ou gérarhdite) sera de préférence à éviter).

La présente invention a ainsi pour objet un procédé de préparation d'un précurseur , de préférence hydroxycarbonaté, de catalyseur contenant du cuivre, de l'aluminium et du zinc, au moins 50%, de préférence au moins 65% et, de manière encore plus préférée, au moins 85% en poids dudit précurseur étant formé par un mélange d'une phase appelée rodérite constituée de cuivre, d'aluminium et de zinc et ayant un rapport atomique $\frac{Cu+Zn}{Al}$ compris entre 3,5 et 16, de préférence entre 4 et 13 et un rapport atomique Zn/Al compris entre 1,6 et 6, de préférence entre 2 et 5, et d'une phase appelée prespinelle microcristallisée, qui est un autre objet de l'invention, constituée de cuivre, d'aluminium et de zinc et ayant un rapport atomique $\frac{Cu+Zn}{Al}$ compris entre 0,20 et 2, 10 , de préférence entre 0,25 et 1,50 et un rapport atomique Zn/Al compris entre 0,10 et 1,50 ,de préférence entre 0,5 et 1,20. Ledit procédé est caractérisé en ce que ledit précurseur résulte du mélange (ou mixtion) de deux précurseurs ternaires, de préférence hydroxycarbonatés, comprenant chacun du cuivre, de l'aluminium et du zinc, l'un desdits précurseurs ternaires contenant au moins 50%, de préférence au moins 65% et, de manière encore plus préférée, au moins 85% en poids de phase rodérite et l'autre desdits précurseurs ternaires contenant au moins 50% de préférence au moins 65% et, de manière encore plus préférée, au moins 85% en poids de phase prespinelle microcristallisée, ledit mélange (ou ladite mixtion) ayant lieu entre, d'une part, l'un des précurseurs ternaires obtenus à l'issue de l'une des étapes 1/ à 4/ d'un procédé P comprenant les étapes suivantes :

1/ réaction de coprécipitation au sein d'un réacteur contenant préalablement de l'eau (par exemple bipermutée), entre une solution aqueuse d'au moins un composé d'un constituant choisi dans le groupe formé par les métaux alcalins (tels que le sodium, le potassium) et l'ion ammonium, ledit composé étant choisi dans le groupe formé par les carbonates, les hydrogénocarbonates et les hydroxydes, et une solution aqueuse contenant, sous forme de composés choisis dans le groupe formé par les sels solubles et les complexes solubles (de préférence solubles en milieu acide), du cuivre, de l'aluminium, et du zinc dans des proportions telles que le rapport atomique $\frac{Cu + Zn}{Al}$ est compris entre 3,5 et 16, de préférence entre 4 et 13 et le rapport atomique Zn/Al est compris entre 1,6 et 6, de préférence entre 2 et 5, ladite réaction ayant lieu à un pH habituellement compris entre 6,3 et 7,3, de préférence entre 6,7 et 7,1, à une température généralement comprise entre 70 et 90°C, de préférence entre 75 et 85°C, avec un temps de résidence ou temps de séjour moyen dans le réacteur compris entre 0,1 et 60 minutes, de préférence entre 10 et 45 minutes (ce temps de séjour moyen est défini comme l'inverse du rapport du débit volumique total (en litres/minutes) des solutions injectées dans le réacteur, au volume dudit réacteur (en litres)),
2/ filtration, puis lavage à l'eau du produit obtenu à l'issue de l'étape 1/,
3/ sèchage du produit obtenu à l'issue de l'étape 2/, entre 50 et 200°C,
4/ calcination du produit obtenu à l'issue de l'étape 3/, entre 250 et 500°C, de préférence entre 280 et 380°C;

et, d'autre part, l'un des précurseurs ternaires obtenus à l'issue de l'une des étapes 1/ à 4/ d'un procédé P' comprenant les étapes suivantes:

1/ réaction de coprécipitation au sein d'un réacteur contenant préalablement de l'eau (par exemple bipermutée), entre une solution aqueuse d'au moins un composé d'un constituant choisi dans le groupe formé par les métaux alcalins (tels que le sodium, le potassium) et l'on ammonium, ledit composé étant choisi dans le groupe formé par les carbonates, les hydrogénocarbonates et les hydroxydes, et une solution aqueuse contenant, sous forme de composés choisis dans le groupe formé par les sels solubles et les complexes solubles (de préférence solubles en milieu acide), du cuivre, de l'aluminium et du zinc dans des proportions telles que le rapport atomique $\frac{Cu + Zn}{Al}$ est compris entre 0,20 et 2,10, de préférence entre 0,25 et 1,50, et le rapport atomique Zn/Al est compris entre 0,10 et 1,50, de préférence entre 0,15 et 1,20, ladite réaction ayant lieu à un pH habituellement compris entre 6,3 et 7,3, de préférence entre 6,7 et 7,1, à une température généralement comprise entre 45 et 65°C, de préférence entre 50 et 60°C, avec un temps de résidence ou temps de séjour moyen dans le réacteur compris entre 0,1 et 60 minutes, de préférence entre 10 et 45 minutes,
2/ filtration, puis lavage à l'eau du produit obtenu à l'issue de l'étape 1/,
3/ séchage du produit obtenu à l'issue de l'étape 2/, entre 50 et 200°C,
4/ calcination du produit obtenu à l'issue de l'étape 3/, entre 250 et 500°C, de préférence entre 280 et 380°C.

Ledit mélange (ou ladite mixtion) est suivi(e) :

- d'une filtration et d'un lavage à l'eau, puis d'un séchage entre 50 et 200°C, puis d'une calcination entre 250 et 500°C, de préférence entre 280 et 380°C, si au moins l'un des deux précurseurs ternaires qui ont été mélangés a été préalablement obtenu à l'issue de l'étape 1/ de l'un des procédés P et P' décrits ci-dessus,
- d'un séchage entre 50 et 200°C, puis d'une calcination entre 250 et 500°C, de préférence entre 280 et 380°C, si l'un des deux précurseurs ternaires qui ont été mélangés a été préalablement obtenu à l'issue de l'étape 2/ de l'un des procédés P et P' décrits ci-dessus, l'autre des deux précurseurs ternaires qui ont été mélangés ayant été préalablement obtenu à l'issue de l'une des étapes 2/ à 4/ de l'un des procédés P et P' décrits ci-dessus, ou
- d'une calcination entre 250 et 500°C, de préférence entre 280 et 380°C, si les deux précurseurs ternaires qui ont été mélangés ont été préalablement obtenus à l'issue de l'une des étapes 3/ et 4/ de l'un des procédés P et P' décrits ci-dessus.

Ainsi le procédé selon l'invention peut être mis en oeuvre selon de nombreuses variantes, ci-dessous référencées de A à J (pour chaque variante, on énumère les étapes que comprend ledit procédé):

A:

a/ réaction de coprécipitation selon l'étape 1/ du procédé P décrit précédemment,
b/ filtration puis lavage à l'eau du produit obtenu à l'issue de l'étape a/,
c/ réaction de coprécipitation selon l'étape 1/ du procédé P' décrit précédemment,
d/ filtration puis lavage à l'eau du produit obtenu à l'issue de l'étape c/,

e/ mélange (ou mixtion) du produit obtenu à l'issue de l'étape b/ avec le produit obtenu à l'issue de l'étape d/,

f/ séchage du produit obtenu à l'issue de l'étape e/, entre 50 et 200°C,

g/ calcination du produit obtenu à l'issue de l'étape f/ entre 250 et 500°C, de préférence entre 280 et 380°C.

B :

a/ réaction de coprécipitation selon l'étape 1/ du procédé P décrit précédemment,

b/ filtration puis lavage à l'eau du produit obtenu à l'issue de l'étape a/,

c/ séchage du produit obtenu à l'issue de l'étape b, entre 50 et 200°C,

d/ réaction de coprécipitation selon l'étape 1/ du procédé P' décrit précédemment,

e/ filtration puis lavage à l'eau du produit obtenu à l'issue de l'étape d/,

f/ séchage du produit obtenu à l'issue de l'étape e/, entre 50 et 200°C,

g/ mélange (ou mixtion) du produit obtenu à l'issue de l'étape c/ avec le produit obtenu à l'issue de l'étape f/,

h/ calcination du produit obtenu à l'issue de l'étape g/ entre 250 et 500°C, de préférence entre 280 et 380°C.

C:

a/ réaction de coprécipitation selon l'étape 1/ du procédé P décrit précédemment,

b/ filtration puis lavage à l'eau du produit obtenu à l'issue de l'étape a/,

c/ séchage du produit obtenu à l'issue de l'étape b/, entre 50 et 200°C,

d/ calcination du produit obtenu à l'issue de l'étape c/, entre 250 et 500°C, de préférence entre 280 et 380°C,

e/ réaction de coprécipitation selon l'étape 1/ du procédé P' décrit précédemment,

f/ filtration puis lavage à l'eau du produit obtenu à l'issue de l'étape e/, g/séchage du produit obtenu à l'issue de l'étape f/, entre 50 et 200°C,

h/ calcination du produit obtenu à l'issue de l'étape g/, entre 250 et 500°C, de préférence entre 280 et 380°C,

i/ mélange (ou mixtion) du produit obtenu à l'issue de l'étape d/ avec le produit obtenu à l'issue de l'étape h/,

j/ calcination du produit obtenu à l'issue de l'étape i/, entre 250 et 500°C, de préférence entre 280 et 380°C.

D :

a/ réaction de coprécipitation selon l'étape 1/ du procédé P décrit précédemment,

b/ filtration puis lavage à l'eau du produit obtenu à l'issue de l'étape a/,

c/ séchage du produit obtenu à l'issue de l'étape b/, entre 50 et 200°C,

d/ réaction de coprécipitation selon l'étape 1/ du procédé P' décrit précédemment,

e/ filtration puis lavage à l'eau du produit obtenu à l'issue de l'étape d/,

f/ mélange (ou mixtion) du produit obtenu à l'issue de l'étape c/ avec le produit obtenu à l'issue de l'étape e/,

g/ séchage du produit obtenu à l'issue de l'étape f/, entre 50 et 200°C,

h/ calcination du produit obtenu à l'issue de l'étape g/, entre 250 et 500°C, de préférence entre 280 et 380°C.

E :

a/ réaction de coprécipitation selon l'étape 1/ du procédé P décrit précédemment.

b/ filtration puis lavage à l'eau du produit obtenu à l'issue de l'étape a/,

c/ séchage du produit obtenu à l'issue de l'étape b/, entre 50 et 200°C,

d/ calcination du produit obtenu à l'issue de l'étape c/, entre 250 et 500°C, de préférence entre 280 et 380°C,

e/ réaction de coprécipitation selon l'étape 1/ du procédé P' décrit précédemment,

f/ filtration puis lavage à l'eau du produit obtenu à l'issue de l'étape e/,

g/ mélange (ou mixtion) du produit obtenu à l'issue de l'étape d/ avec le produit obtenu à l'issue de l'étape f/,

h/ séchage du produit obtenu à l'issue de l'étape g/, entre 50 et 200°C,

i/ calcination du produit obtenu à l'issue de l'étape h/, entre 250 et 500°C, de préférence entre 280 et 380°C.

F :

a/ réaction de coprécipitation selon l'étape 1/ du procédé P' décrit précédemment,

b/ filtration puis lavage à l'eau du produit obtenu à l'issue de l'étape a/,

c/ séchage du produit obtenu à l'issue de l'étape b/, entre 50 et 200°C,

d/ réaction de coprécipitation selon l'étape 1/ du procédé P décrit précédemment.

e/ filtration puis lavage à l'eau du produit obtenu à l'issue de l'étape d/,

f/ mélange (ou mixtion) du produit obtenu à l'issue de l'étape c/ avec le produit obtenu à l'issue de l'étape e/,

g/ séchage du produit obtenu à l'issue de l'étape f/, entre 50 et 200°C,

h/ calcination du produit obtenu à l'issue de l'étape g/, entre 250 et 500°C, de préférence entre 280 et 380°C.

G :

a/ réaction de coprécipitation selon l'étape 1/ du procédé P' décrit précédemment,

b/ filtration puis lavage à l'eau du produit obtenu à l'issue de l'étape a/,

c/ séchage du produit obtenu à l'issue de l'étape b/, entre 50 et 200°C,

d/ calcination du produit obtenu à l'issue de l'étape c/, entre 250 et 500°C, de préférence entre 280 et 380°C,

e/ réaction de coprécipitation selon l'étape 1/ du procédé P décrit précédemment,

f/ filtration puis lavage à l'eau du produit obtenu à l'issue de l'étape e/,

g/ mélange (ou mixtion) du produit obtenu à l'issue de l'étape d/ avec le produit obtenu à l'issue de l'étape f/,

h/ séchage du produit obtenu à l'issue de l'étape g/, entre 50 et 200°C,

i/ calcination du produit obtenu à l'issue de l'étape h/, entre 250 et 500°C, de préférence entre 280 et 380°C.

H :

a/ réaction de coprécipitation selon l'étape 1/ du procédé P décrit précédemment,

b/ filtration puis lavage à l'eau du produit obtenu à l'issue de l'étape a/,

c/ séchage du produit obtenu à l'issue de l'étape b/, entre 50 et 200°C,

d/ calcination du produit obtenu à l'issue de l'étape c/, entre 250 et 500°C, de préférence entre 280 et 380°C,

e/ réaction de coprécipitation selon l'étape 1/ du procédé P' décrit précédemment,

f/ filtration puis lavage à l'eau du produit obtenu à l'issue de l'étape e/,

g/ séchage du produit obtenu à l'issue de l'étape f/, entre 50 et 200°C,

h/ mélange (ou mixtion) du produit obtenu à l'issue de l'étape d/ avec le produit obtenu à l'issue de l'étape g/,

i/ calcination du produit obtenu à l'issue de l'étape h/, entre 250 et 500°C, de préférence entre 280 et 380°C.

I :

a/ réaction de coprécipitation selon l'étape 1/ du procédé P' décrit précédemment,

b/ filtration puis lavage à l'eau du produit obtenu à l'issue de l'étape a/,

c/ séchage du produit obtenu à l'issue de l'étape b/, entre 50 et 200 °C,

d/ calcination du produit obtenu à l'issue de l'étape c/, entre 250 et 500°C, de préférence entre 280 et 380°C,

e/ réaction de coprécipitation selon l'étape 1/, du procédé P décrit précédemment,

f/ filtration puis lavage à l'eau du produit obtenu à l'issue de l'étape e/,

g/ séchage du produit obtenu à l'issue de l'étape f/, entre 50 et 200°C,

h/ mélange (ou mixtion) du produit obtenu à l'issue de l'étape d/ avec le produit obtenu à l'issue de l'étape g/,

i/ calcination du produit obtenu à l'issue de l'étape h/, entre 250 et 500°C, de préférence entre 280 et 380°C.

J :

a/ réaction de coprécipitation selon l'étape 1/ du procédé P décrit précédemment,

b/ réaction de coprécipitation selon l'étape 1/ du procédé P' décrit précédemment,

c/ mélange (ou mixtion) du produit obtenu à l'issue de l'étape a/ avec le produit obtenu à l'issue de l'étape b/,

d/ filtration puis lavage à l'eau du produit obtenu à l'issue de l'étape c/,

e/ séchage du produit obtenu à l'issue de l'étape d/, entre 50 et 200°C,

f/ calcination du produit obtenu à l'issue de l'étape e/, entre 250 et 500°C, de préférence entre 280 et 380°C.

Eventuellement, dans les procédés selon l'invention, au moins une, de préférence chaque, étape de séchage est immédiatement suivie d'un broyage du produit obtenu à l'issue de ladite étape de séchage, afin d'obtenir un produit sous forme de poudre dont la granulométrie est, de préférence, inférieure à 2 $\mu$m (2.10$^{-6}$m) (le broyage s'effectuant alors entre ladite étape de séchage et l'étape suivant cette dite étape de séchage). On peut effectuer un tel broyage entre l'étape de séchage et une étape de mélange.

Il peut être avantageux que, dans les procédés selon l'invention qui comportent deux (respectivement trois) étapes de calcination, la première (respectivement au moins une des deux premières) étape de calcination soit immédiatement suivie d'un broyage du produit obtenu à l'issue de ladite première étape de calcination (respectivement de ladite au moins une des deux premières étapes de calcination), afin d'obtenir un produit sous forme de poudre dont la granulométrie est, de préférence, inférieure à 2 $\mu$m (2.10$^{-6}$m) (le broyage s'effectuant alors entre ladite étape de calcination et l'étape suivant cette dite étape de calcination). Néanmoins, un tel broyage peut être prévu immédiatement avant au moins une étape de calcination.

Le broyage peut s'effectuer par exemple dans un broyeur de type ALPINE. Parmi les variantes A à J décrites précédemment, les variantes A, B,C et J sont préférées.

Dans lesdites variantes A à J, l'étape de mélange de deux produits (ou précurseurs ternaires) consiste par exemple en un mélange de ces deux produits au moyen d'une turbine présentant habituellement un fort taux de cisaillement, telle qu'une turbine de type STARO (mise en suspension dans de l'eau distillée des deux produits puis agitation vigoureuse au moyen de ladite turbine); cette étape de mélange ou mixtion peut également s'effectuer dans tout malaxeur approprié, par exemple dans un malaxeur en V.

Le mélange des deux précurseurs ternaires peut également consister à précipiter l'un desdits précurseurs ternaires dans un réacteur en présence de l'autre précurseur ternaire introduit en suspension dans l'eau dans ledit réacteur.

En effet, la présente invention a également pour objet un procédé de préparation d'un précurseur, de préférence hydroxycarbonaté, de catalyseur contenant du cuivre, de l'aluminium et du zinc, au moins 50%, de préférence au moins 65% et, de manière encore plus préférée, au moins 85% en poids dudit précurseur étant formé par un mélange d'une phase appelée rodérite constituée de cuivre, d'aluminium et de zinc et ayant un rapport atomique $\frac{Cu+Zn}{Al}$ compris entre 3,5 et 16, de préférence entre 4 et 13 et un rapport atomique Zn/Al compris entre 1,6 et 6, de préférence entre 2 et 5, et d'une phase appelée prespinelle microcristallisée constituée de cuivre, d'aluminium et de zinc et ayant un rapport atomique $\frac{Cu+Zn}{Al}$ compris entre 0,20 et 2,10, de préférence entre 0,25 et 1,50 et un rapport atomique Zn/Al compris entre 0,10 et 1,50, de préférence entre 0,15 et 1,20; ledit procédé est caractérisé en ce que ledit précurseur résulte du mélange de deux précurseurs ternaires comprenant chacun du cuivre, de l'aluminium et du zinc, l'un desdits précurseurs ternaires contenant au moins 50%, de préférence au moins 65% et, de manière encore plus préférée, au moins 85% en poids de phase rodérite et l'autre desdits précurseurs ternaires contenant au moins 50%, de préférence au moins 65% et, de manière encore plus préférée, au moins 85% en poids de phase prespinelle microcristallisée, et en ce qu'il comprend les étapes suivantes :

a/ étape correspondant à l'étape 1/ du procédé P (respectivement P') décrit précédemment,

b/ filtration, puis lavage à l'eau du produit obtenu à l'issue de l'étape a/,

c/ étape de coprécipitation correspondant à l'étape 1/ du procédé P' (respectivement P) décrit précédemment, excepté le fait que, en plus des deux solutions employées pour la coprécipitation , on a également introduit dans le réacteur, de préférence simultanément à l'introduction des deux dites solutions, de l'eau (par exemple bipermutée) dans laquelle se trouve en suspension le produit obtenu à l'issue de l'étape b/, de manière à obtenir un mélange de deux produits, le premier ayant été obtenu à l'issue de l'étape b/ (c'est le produit mis en suspension dans l'eau), le second ayant précipité au cours de la réaction de coprécipitation constituant cette étape c/ sur le premier,

d/filtration, puis lavage à l'eau du mélange de produits obtenu à l'issue de l'étape c/,

e/ séchage du produit obtenu à l'issue de l'étude d/, entre 50 et 200°C,

f/ calcination du produit obtenu à l'issue de l'étape e/, entre 250 et 500°C, de préférence entre 280 et 380°C.

Dans chaque réaction de coprécipitation contenue dans les procédés selon l'invention, la solution aqueuse contenant du cuivre, de l'aluminium et du zinc est par exemple une solution aqueuse d'oxydes solubles, d'hydroxydes, de carbonates et/ou d'hydroxycarbonates, solubles de préférence en milieu acide, du cuivre, de l'aluminium et du zinc (par

exemple $CuCO_3$-$Cu(OH)_2$, $ZnCO_3$,$Zn(OH)_2$, $Al(OH)_3$, les nitrates, oxalates, tartrates, citrates, acétates, acétylacétonates),éventuellement de complexes aminés (solubles en milieu ammoniacal) du cuivre et du zinc, les nitrates étant les sels solubles qui sont le plus souvent mis en oeuvre dans ladite réaction de coprécipitation.

Ladite solution aqueuse contient généralement 0,7 à 2 atomes gramme environ de l'ensemble des métaux (Cu+Al+Zn) par litre. L'autre solution mise en jeu dans la réaction de coprécipitation contient généralement 0,7 à 3,2 atomes gramme environ de cations alcalins et/ou d'ions ammonium par litre.

Toute technique et dispositif connu de l'homme du métier concernant la coprécipitation peuvent être utilisés; ainsi, les deux (ou trois) solutions mises en jeu dans chaque réaction de coprécipitation peuvent être introduites dans le réacteur dans n'importe quel ordre; de préférence, on ajoute les deux (ou trois) solutions de façon simultanée, et en régulant leurs débits par le pH mesuré dans la zone réactionnelle, dans un réacteur comportant, de manière avantageuse, un système d'agitation efficace.

On opère de préférence en continu; le volume utile du réacteur peut varier de quelques litres à environ 100 litres et le produit obtenu à l'issue de la réaction de coprécipitation est généralement récupéré en continu puis envoyé sur un filtre, par exemple un filtre presse ou un filtre rotatif où il est ensuite lavé à l'eau (par exemple bipermutée) une ou plusieurs fois.

Chaque étape de séchage peut être réalisée par tout procédé connu de l'homme du métier. On peut l'effectuer par exemple par atomisation (spray-drying); on obtient alors en général un produit contenant environ 60 à environ 80% en poids d'oxydes potentiels. On peut également effectuer le séchage en étuve, sous balayage d'air, de façon à ramener si nécessaire la teneur pondérale en oxydes potentiels entre environ 60 et 80%. Le séchage s'effectue en général pendant un temps suffisant pour que la teneur pondérale en eau du produit après séchage soit par exemple inférieure à 10%. Lors d'un séchage suivant une étape de filtration et lavage, il est recommandé d'éviter la stagnation du précipité en présence de pressions partielles de vapeur d'eau proches de la pression de vapeur saturante à la température de séchage considérée ; de tels traitements peuvent avoir pour conséquence une déshydratation partielle du précipité avec cristallisation d'oxyde cuivrique en gros cristallites.

Chaque étape de calcination (ou activation thermique) peut être menée en présence d'un gaz inerte contenant généralement 0 à 50% en volume d'oxygène.

La dernière étape de calcination des procédés selon l'invention est en général menée pendant une durée suffisante, par exemple pendant au moins 30 minutes, pour obtenir un produit activé ne renfermant généralement pas plus de 12% en poids de matières volatiles (le taux de matières volatiles est mesuré par exemple par activation en présence d'air d'un poids donné de produit, placé dans une nacelle et calciné à 500-600°C pendant 4 heures).

Les opérations de séchage et d'activation thermique peuvent éventuellement être combinées par utilisation de techniques de grillage-éclair ou de calcination par atomisation (spray-calcination), le produit étant alors pulvérisé dans un courant de gaz de combustion.

On peut éventuellement préparer le précurseur , de préférence hydroxycarbonaté , en n'effectuant qu'une seule réaction de coprécipitation , dite alors coprécipitation mixte, dans laquelle on précipite simultanément dans le même milieu (et non séparemment comme dans les procédés décrits précédemment) les deux précurseurs ternaires, de préférence hydroxycarbonatés ; ce procédé particulier comprend les étapes suivantes :

1/ réaction de coprécipitation mixte au sein d'un réacteur contenant préalablement de l'eau (par exemple bipermutée) entre une solution aqueuse d'au moins un composé d'un constituant choisi dans le groupe formé par les métaux alcalins (tels que le sodium, le potassium) et l'ion ammonium, ledit composé étant choisi dans le groupe formé par les carbonates, les hydrogénocarbonates et les hydroxydes, et une solution aqueuse contenant, sous forme de composés choisis dans le groupe formé par les sels solubles et les complexes solubles (de préférence solubles en milieu acide), du cuivre, de l'aluminium et du zinc dans des proportions telles que le rapport atomique $\frac{Cu + Zn}{Al}$ est compris entre 0,9 et 5,1 , de préférence entre 1,1 et 4,6, et le rapport atomique Zn/Al est compris entre 0,7 et 3,1, de préférence entre 0,9 et 2,6 , ladite réaction ayant lieu à un pH habituellement compris entre 6,3 et 7,3, de préférence entre 6,7 et 7,1, à une température généralement comprise entre 45 et 90°C, avec un temps de résidence ou temps de séjour moyen dans le réacteur compris entre 0,1 et 60 minutes, de préférence entre 10 et 45 minutes ,

2/ filtration, puis lavage à l'eau du produit obtenu à l'issue de l'étape 1/,

3/ séchage du produit obtenu à l'issue de l'étape 2/, entre 50 et 200°C,

4/ calcination du produit obtenu à l'issue de l'étape 3/, entre 250 et 500°C, de préférence 280 et 380°C.

Les proportions relatives en phase rodérite et en phase prespinelle microcristallisée dudit précurseur ainsi préparé par ce dernier procédé pourront être modifiées, par exemple en ajustant la température opératoire de l'opération unitaire de coprécipitation (étape 1/), comprise entre 45 et 90°C.

Ainsi, pour une température comprise entre 45 et 65°C, ledit précurseur sera généralement riche en phase prespinelle (la phase prespinelle représentant alors par exemple au moins 60% en poids de l'ensemble (phase prespinelle + phase rodérite)); pour une température comprise entre 70 et 90°C, ledit précurseur sera généralement riche en phase rodérite (la phase rodérite représentant alors par exemple au moins 60% en poids de l'ensemble (phase prespinelle + phase rodérite)).

A partir du précurseur , de préférence hydroxycarbonaté, préparé selon un des procédés de la présente invention, on peut fabriquer un catalyseur, par exemple selon les étapes suivantes :

1/ broyage éventuel dudit précurseur préparé selon un des procédés décrits précédemment, en général jusqu'à une granulométrie inférieure à 1 mm, de préférence à 0,5 mm,

2/ mise en forme du produit obtenu à l'issue de l'étape 1/, par tout procédé connu de l'homme de l'art (extrusion, dragéification, coagulation en gouttes, etc), incluant éventuellement un mélange dudit produit à raison de 0,5-5% de son poids avec au moins un composé choisi dans le groupe formé par le graphite, l'acide stéarique, les stéarates, et éventuellement un adjuvant de porosité choisi parmi la cellulose et les poudres d'origine végétale en contenant, les carbonates d'ammonium, les filtres textiles combustibles et le naphtalène, ledit produit pouvant être alors éventuellement pastiller en cylindres pleins de diamètres 2-6 mm ou en cylindres toriques de diamètres extérieur 3-6 mm et intérieur 1-4 mm et de hauteur 2-6 mm,

3/ calcination du produit obtenu à l'issue de l'étape 2/, en général entre 250 et 500°C, de préférence entre 280 et 380°C.

Tout procédé de préparation d'un catalyseur à partir du précurseur , de préférence hydroxycarbonaté, comprenant par exemple une étape de broyage et de mise en forme, une étape finale éventuelle de calcination en général entre 250 et 500°C, de préférence entre 280 et 380°C, pourra cependant être utilisée.

Ledit catalyseur ainsi préparé renferme du cuivre , du zinc et de l'aluminium dans les proportions pondérales suivantes, rapportées au poids total des métaux présents dans ledit catalyseur :

- 15 à 80%, de préférence 20 à 70% de cuivre,
- 4 à 50%, de préférence 4 à 40% d'aluminium, et
- 10 à 70%, de préférence 20 à 60%, de zinc.

Ledit catalyseur peut être utilisé dans les procédés mettant en jeu une réaction équilibrée des oxydes de carbone CO et $CO_2$ avec l'hydrogène du fait de ses excellentes activité, sélectivité et stabilité, et notamment dans les procédés de production de méthanol (ou d'éthanol) à partir d'oxydes de carbone CO et $CO_2$ et d'hydrogène, et dans les procédés de décomposition d'au moins un alcool primaire de $C_1$ à $C_5$ (en particulier méthanol et/ou éthanol) en oxydes de carbone CO et $CO_2$ et hydrogène.

Préalablement à son utilisation dans lesdits procédés, ledit catalyseur est de préférence préréduit par un mélange de gaz inerte (azote par exemple) et d'au moins un composé réducteur choisi dans le groupe formé par l'hydrogène, le monoxyde de carbone, les alcools et les aldéhydes en $C_1$ et $C_2$, le rapport molaire composé réducteur/(composé réducteur + gaz inerte) étant de 0,001 : 1 à 1 : 1.

La température de réduction varie généralement de 100 à 300°C environ, de préférence entre 140 et 260°C environ; la pression totale est habituellement comprise entre 0,1 et 10 MPa environ et, de préférence, entre 0,1 et 6 MPa environ; la vitesse volumétrique horaire est habituellement comprise entre $10^2$ et $4.10^4$ heure$^{-1}$ environ et de préférence entre $5.10^2$ et $10^4$ heure$^{-1}$ (Température et Pression Normales (TPN)).

La réduction est d'abord menée, par exemple entre environ 140 et environ 160°C, en présence du mélange réducteur précité et avec un rapport molaire gaz réducteur/(gaz réducteur + gaz inerte) compris entre 0,001 et 0,1 environ et préférentiellement entre 0,005 et 0,05 environ, pendant un temps suffisant pour que les concentrations en gaz réducteur soient les mêmes à l'entrée et à la sortie du réacteur (ce qui prouve que la première étape de réduction est terminée); il peut être avantageux dans une seconde étape d'augmenter la température ainsi qu'éventuellement la concentration en gaz réducteur, et de poursuivre la réduction dans des conditions thermiques plus sévères.

La température de réduction varie alors entre environ 160 et environ 260°C, le rapport molaire gaz réducteur/(gaz réducteur + gaz inerte) est alors compris entre 0,01 et 1 et préférentiellement entre 0,05 et 1, les pressions et vitesse volumétrique horaire restant à l'intérieur des fourchettes précitées.

La réaction de synthèse du méthanol est généralement réalisée dans les conditions opératoires suivantes : la pression est habituellement comprise entre 2 et 15 MPa environ et de préférence entre 4 et 15 MPa environ, le rapport molaire $H_2/(2CO+3CO_2)$ est avantageusement compris entre 0,4 et 10 environ, de préférence entre 0,5 et 4 environ, lorsque la réaction est menée en présence d'une phase gazeuse, et compris de préférence entre 0,5 et 1,5 environ lorsque la réaction est menée en présence d'une phase liquide et d'une phase gazeuse. La température est comprise entre 200 et 300°C environ, de préférence entre 210 et 270°C environ.

La vitesse volumique horaire (exprimée en volume TPN de mélange gazeux par volume de catalyseur et par heure) est usuellement comprise entre 1500 et 60 000 h$^{-1}$ environ et, de préférence, entre 2000 et 20 000 h$^{-1}$ environ.

La réaction de décomposition d'au moins un alcool primaire de $C_1$ à $C_5$ (en particulier méthanol et/ou éthanol) est réalisée dans les conditions opératoires suivantes : la pression est habituellement comprise entre 1 et 6 MPa environ et, de préférence, entre 2 et 5 MPa environ. La température est comprise entre 200 et 320°C environ, de préférence entre 220 et 300°C environ.

La vitesse volumique horaire de la charge (exprimée en litre de charge par litre de catalyseur et par heure) est usuellement comprise entre 0,1 et 5h$^{-1}$ environ et, de préférence, entre 0,5 et 3 h$^{-1}$ environ.

Les exemples suivants illustrent l'invention sans en limiter la portée.

Dans les exemples 1 à 5 ci-dessous, les précurseurs rodérite et prespinelle sont éventuellement préparés dans deux réacteurs séparés (réacteurs 1 et 2). A l'issue de l'étape de coprécipitation on obtient donc les précurseurs respectivement appelés R1 et P1 (étape 1), qui sont lavés (étape 2) pour obtenir les précurseurs respectivement appelés R2 et P2, et éventuellement mélangés (précurseur RP2). Après l'étape de séchage (étape 3) les précurseurs rodérite et prespinelle respectivement appelés R3 et P3, ou le précurseur issu du séchage du produit RP2 (précurseur RP3), sont éventuellement calcinés (étape 4), séparément pour obtenir les produits référencés R4 et P4, ou après mélange de R3 et P3 (produit RP4). Cette calcination est éventuellement suivie d'une étape de mise en forme (étape 5, produits R5, P5, RP5).

(on entend par précurseur rodérite le précurseur contenant la phase rodérite et par précurseur prespinelle le précurseur contenant la phase prespinelle).

### Exemple 1: Catalyseur A (selon l'invention)

La préparation du précurseur rodérite R2 est tout d'abord opérée comme suit:

- On dissout 253,61 g de nitrate de cuivre trihydraté (1,05 at.g Cu), 44,61 g de nitrate d'aluminium nonahydraté (0,12 at.g Al), 140,48 g de zinc hexahydraté (0,47 at.g Zn) dans 1,5 litres d'eau bipermutée, afint d'obtenir une solution (solution I) contenant 1,09 at.g de métaux par litre.
- On dissout séparément 226,14 g de carbonate dissodique dans 4 litres d'eau. On obtient une solution II contenant 1,07 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 75 à 85 °C. La température est maintenue entre 75 et 85 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit R1) est récupéré en continu dans un autre réacteur, filtré et lavé par trois fois 12 litres d'eau. A l'issue de cette étape 2, on dispose du précurseur R2 contenant au moins 85 % en poids de phase rodérite.

On prépare ensuite le précurseur prespinelle P2 de la façon suivante:

- On dissout 98,73 g de nitrate de cuivre trihydraté (0,41 at.g), 306,5 g de nitrate d'aluminium nonahydraté (0,82 at.g Al), 229,57 g de zinc hexahydraté (0,77 at.g Zn) dans 2 titres d'eau bipermutée, afin d'obtenir une solution (solution I) contenant 1 at.g de métaux par litre.
- On dissout séparément 275,31 g de carbonate dissodique dans 4 litres d'eau. On obtient une solution II contenant 1,03 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 50 à 60 °C. La température est maintenue entre 50 et 60 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit P1) est récupéré en continu dans un autre réacteur, filtré et lavé par trois fois 12 litres d'eau. A l'issue de cette étape 2, on dispose du précurseur P2 contenant au moins 85% en poids de phase rodérite.

Le précurseur du catalyseur est obtenu ensuite par malaxage des deux précurseurs R2 et P2 au moyen d'une turbine de type STARO pendant 15 minutes, puis le produit malaxé est remis en suspension dans environ 600 cm$^3$ d'eau bipermutée et séché dans un atomiseur de type BUCHI (température de sortie = 150 °C, temps de séjour moyen t = 1,5 seconde) pour obtenir le produit RP3 contenant 70 % en poids d'oxydes Le catalyseur est alors obtenu par calcination sous air à 300 °C (produit RP4). La teneur en matières volatiles résiduelles est alors de 5 %.

La poudre obtenue est finalement mélangée avec 2 % en poids de graphite, pastillée en cyclindres de 2,4 mm de diamètre et de 2 mm de hauteur, puis activée à 300 °C sous air. On obtient environ 265 g de catalyseur A.

### Exemple 2 : Catalyseur B (selon l'invention)

La préparation du précurseur rodérite R2 est tout d'abord opérée comme suit:

- On dissout 173,56 g de nitrate de cuivre trihydraté (0,72 at.g Cu), 127,96 g de nitrate d'aluminium nonahydraté (0,34 at.g Al), 213,65 g de zinc hexahydraté (0,72 a.t g Zn) dans 1,5 litres d'eau bipermutée, afint d'obtenir une solution (solution I) contenant 1,18 at.g de métaux par litre.
- On dissout séparément 245,0 g de carbonate dissodique dans 4 litres d'eau. On obtient une solution II contenant 1,16 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 75 à 85 °C. La température est maintenue entre 75 et 85 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit R1) est récupéré en continu dans un autre réacteur, filtré et lavé par trois fois 12 litres d'eau. A l'issue de cette étape 2, on dispose du précurseur R2 contenant au moins 85 % en poids de phase rodérite.

On prépare ensuite le précurseur prespinelle P2 de Ta façon suivante:

- On dissout 23,9 g de nitrate de cuivre trihydraté (0,1 at.g), 316 g de nitrate d'aluminium nonahydraté (0,84 at.g Al), 40,7 g de zinc hexahydraté (0,13 at.g Zn) dans 1 litre d'eau bipermutée, afin d'obtenir une solution (solution I) contenant 1,08 at.g de métaux par litre.
- On dissout séparément 148,65 g de carbonate dissodique dans 2 litres d'eau. On obtient une solution II contenant 2,8 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 50 à 60°C. La température est maintenue entre 50 et 60 °C pendant toute la durée de la précipitation. Le temps de résidence est de l'ordre de 40 mn.

Les débits sont régulés par le pH, qui varie entre 6,7 et 7,1 pendant toute la durée de la réaction. Le produit de la réaction (produit P1) est récupéré en continu dans un autre réacteur, filtré et lavé par trois fois 12 litres d'eau. A l'issue de cette étape 2, on dispose du précurseur P2 contenant au moins 85% en poids de phase prespinelle.

Le précurseur du catalyseur est obtenu ensuite par malaxage pendant 20 minutes des deux précurseurs R2 et P2 au moyen d'une turbine de type STARO pendant 15 minutes, puis le produit malaxé est remis en suspension dans environ 600 $cm^3$ d'eau bipermutée et séché dans un atomiseur de type BUCHI (température de sortie = 150 °C, temps de séjour moyen t = 1,5 seconde) pour obtenir le produit RP3 contenant 70 % en poids d'oxydes. Le catalyseur est alors obtenu par calcination sous air à 300 °C (produit RP4). La teneur en matières volatiles résiduelles est alors de 4 %.

La poudre obtenue est finalement mélangée avec 2 % en poids de graphite, pastillée en cyclindres de 2,4 mm de diamètre et de 2 mm de hauteur, puis activée à 300 °C sous air. On obtient environ 195 g de catalyseur B.

**Exemple 3: Catalyseur C** (selon l'invention)

La préparation du précurseur rodérite R3 est tout d'abord opérée comme suit:

- On dissout 173,56 g de nitrate de cuivre trihydraté (0,72 at.g Cu), 127,96 g de nitrate d'aluminium nonahydraté (0,34 at.g Al), 213,65 g de zinc hexahydraté (0,72 at.g Zn) dans 1,5 litres d'eau bipermutée, afint d'obtenir une solution (solution I) contenant 1,18 at.g de métaux par litre.
- On dissout séparément 245,01 g de carbonate dissodique dans 4 litres d'eau. On obtient une solution II contenant 1,16 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 75 à 85 °C. La température est maintenue entre 75 et 85 °C pendant toute la durée de la précipitation. Le temps de résidence est de l'ordre de 35 mn.

Les débits sont régulés parle pH, qui varie entre 6,7 et 7,1 pendant toute la durée de la réaction. Le produit de la réaction (produit R1) est récupéré en continu dans un autre réacteur, filtré et lavé par trois fois 12 litres d'eau. A l'issue de cette étape 2, on dispose du précurseur R2 contenant au moins 85 % en poids de phase rodérite. Le précurseur est ensuite remis en suspension dans environ 250 $cm^3$ d'eau bipermutée, puis séché dans un atomiseur de type BUCHI (température de sortie = 145 °C, temps de séjour moyen t = 1,8 seconde). Le produit R3 obtenu contient environ 72 % d'oxydes.

On prépare ensuite le précurseur prespinelle P3 de la façon suivante:

- On dissout 23,9 g de nitrate de cuivre trihydraté (0,1 at.g), 316 g de nitrate d'aluminium nonahydraté (0,84 at.g Al), 40,7 g de zinc hexahydraté (0,13 at.g Zn) dans 1 litre d'eau bipermutée, afin d'obtenir une solution (solution I) contenant 1,08 at.g de métaux par litre.
- On dissout séparément 148,65 g de carbonate dissodique dans 2 litres d'eau. On obtient une solution II contenant 2,8 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 50 à 60 °C. La température est maintenue entre 50 et 60 °C pendant toute la durée de la précipitation. Le temps de résidence est de l'ordre de 40 mn.

Les débits sont régulés par le pH, qui varie entre 6,7 et 7,1 pendant toute la durée de la réaction. Le produit de la réaction (produit P1) est récupéré en continu dans un autre réacteur, filtré et lavé par trois fois 12 litres d'eau. A l'issue de cette étape 2, on dispose du précurseur P2 contenant au moins 85 % en poids de phase prespinelle. Le précurseur est ensuite remis en suspension dans environ 150 cm3 d'eau, puis séché dans un atomiseur de type BUCHI (température de sortie = 145 °C, temps de séjour moyen t = 1,6 seconde). Le produit P3 obtenu contient environ 68 % d'oxydes.

Les deux précurseurs R3 et P3 sont mélanges pendant 2 heures dans un mélangeur de poudres en V, puis le mélange obtenu est broyé à une granulométrie inférieure à 1 micron dans un broyeur de type Alpine où le produit est recyclé 3 fois. Le mélange obtenu est calciné à 300 °C sous air (produit RP4). La teneur en matières volatiles résiduelles est alors de 4 %.

La poudre RP4 obtenue est finalement mélangée avec 2 % en poids de graphite, pastillée en cyclindres de 2,4 mm de diamètre et de 2 mm de hauteur, puis activée à 300 °C sous air. On obtient environ 195 g de catalyseur C.

**Exemple 4: Catalyseur D** (selon l'invention)

La préparation du produit R4 issu du précurseur rodérite est tout d'abord opérée comme suit:

- On dissout 182,31 g de nitrate de cuivre trihydraté (0,75 at.g Cu), 80,18 g de nitrate d'aluminium nonahydraté (0,21 at.g Al), 252,47 g de zinc hexahydraté (0,85 at.g Zn) dans 1,5 litres d'eau bipermutée, afint d'obtenir une solution (solution I) contenant 1,21 at.g de métaux par litre.
- On dissout séparément 250,42 g de carbonate dissodique dans 4 litres d'eau. On obtient une solution II contenant 1,18 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 75 à 85 °C. La température est maintenue entre 75 et 85 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit R1) est récupéré en continu dans un autre réacteur, filtré et lavé par trois fois 12 litres d'eau. A l'issue de cette étape 2, on dispose du précurseur R2 contenant au moins 85 % en poids de phase rodérite.Le produit R2 est ensuite remis en suspension et séché par atomisation à 135 °C pendant 2 secondes (produit R3 à 73 % d'oxydes), puis calciné à 300 °C sous air pour obtenir le produit R4.

On prépare ensuite le produit P4 issu du précurseur prespinelle de la façon suivante:

- On dissout 200,95 g de nitrate de cuivre trihydraté (0,83 at.g), 384,55 g de nitrate d'aluminium nonahydraté (1,03 at.g Al), 67,77 g de zinc hexahydraté (0,23 at.g Zn) dans 2 litres d'eau bipermutée, afin d'obtenir une solution (solution I) contenant 1,05 at.g de métaux par litre.
- On dissout séparément 287,33 g de carbonate dissodique dans 4 litres d'eau. On obtient une solution II contenant 1,35 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 50 à 60 °C. La température est maintenue entre 50 et 60 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit P1) est récupéré en continu dans un autre réacteur, filtré et lavé par trois fois 12 litres d'eau. A l'issue de cette étape 2, on dispose du précurseur P2 contenant au moins 85 % en poids de phase prespinelle.

Le produit P2 est ensuite remis en suspension dans environ 500 cm$^3$ d'eau bipermutée et séché par atomisation à 130 °C pendant 2 secondes (produit P3 à 70 % d'oxydes), puis calciné 2 heures à 300 °C sous air pour obtenir le produit P4.

Les produits P4 et R4 sont alors mélangés pendant 2 heures dans un mélangeur de poudres en V, puis le mélange obtenu est broyé à une granulométrie inférieure à 1 micron dans un broyeur de type Alpine où le produit est recyclé 3 fois.

Le mélange ainsi obtenu est à nouveau calciné 1 heure à 300 °C pour obtenir le produit RP4 contenant 5 % de matières volatiles, qui est mis en forme par mélange avec 2 % en poids de graphite, et pastillage en cylindre de 2,4 mm de diamètre et de 2 mm de hauteur. Cette étape est suivie d'une nouvelle activation à 300 °C sous air. On obtient ainsi environ 277 g de catalyseur D.

**Exemple 5 : Catalyseur E** (selon l'invention)

La préparation du précurseur rodérite R2 est tout d'abord opérée comme suit:

- On dissout 182,31 g de nitrate de cuivre trihydraté (0,75 at.g Cu), 80,18 g de nitrate d'aluminium nonahydraté (0,21 at.g Al), 252,47 g de zinc hexahydraté (0,85 at.g Zn) dans 1,5 litres d'eau bipermutée, afint d'obtenir une solution (solution I) contenant 1,21 at.g de métaux par litre.
- On dissout séparément 250,42 g de carbonate dissodique dans 4 litres d'eau. On obtient une solution II contenant 1,18 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 75 à 85 °C. La température est maintenue entre 75 et 85 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit R1) est récupéré en continu dans un autre réacteur, filtré et lavé par trois fois 12 litres d'eau. A l'issue de cette étape 2, on dispose du précurseur R2 contenant au moins 85% en poids de phase rodérite.

On dissout 200,95 g de nitrate de cuivre trihydraté (0,83 at.g), 384,55 g de nitrate d'aluminium nonahydraté (1,03 at.g Al), 67,77 g de zinc hexahydraté (0,23 at.g Zn) dans 2 litres d'eau bipermutée, afin d'obtenir une solution (solution I) contenant 1,05 at.g de métaux par litre.

On dissout séparément 287,33 g de carbonate dissodique dans 4 litres d'eau. On obtient une solution II contenant 1,35 at.g de sodium par litre.

Le produit R2 préparé ci-dessus est alors remis en suspension dans 1,5 litre d'eau bipermutée (solution (III).

La réaction est menée dans un réacteur de 3 litres opérant en continu. Les trois solutions I, II et III sont injectées simultanément dans le réacteur contenant au préalable 2,8 litres d'eau bipermutée à une température de 50 à 60 °C. La température est maintenue entre 50 et 60 °C pendant toute la durée de la précipitation. Le temps de résidence est de l'ordre de 25 mn.

Les débits sont régulés par le pH, qui varie entre 6,7 et 7,1 pendant toute la durée de la réaction. Le produit de la réaction est récupéré en continu dans un autre réacteur, filtré et lavé par trois fois 12 litres d'eau. A l'issue de cette étape 2, on dispose du précurseur RP2 contenant au moins 85 % de (phase prespinelle + phase rodérite).

Le produit RP2 est ensuite remis en suspension dans environ 0,5 litre d'eau, et séché par atomisation à 130 °C pendant 2 secondes (produit RP3 à 70 % d'oxydes), puis calciné 2 heures à 300 °C sous air pour obtenir le produit RP4 contenant 5 % de matières volatiles, qui est mis en forme par mélange avec 2 % en poids de graphite, et pastillage en cylindre de 2,4 mm de diamètre et de 2 mm de hauteur. Cette étape est suivie d'une nouvelle activation à 300 °C sous air. On obtient ainsi environ 277 g de catalyseur E.

**Exemple 6: Catalyseur F (comparatif)**

La préparation du précurseur rodérite est tout d'abord opérée comme suit:

- On dissout 173,56 g de nitrate de cuivre trihydraté (0,72 at.g Cu), 127,96 g de nitrate d'aluminium nonahydraté (0,34 at.g Al), 213,65 g de zinc hexahydraté (0,72 at.g Zn) dans 1,5 litres d'eau bipermutée, afint d'obtenir une solution (solution I) contenant 1,18 at.g de métaux par litre.
- On dissout séparément 245,01 g de carbonate dissodique dans 4 litres d'eau. On obtient une solution II contenant 1,16 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 75 à 85 °C.

La température est maintenue entre 75 et 85 °C pendant toute la durée de la précipitation. Le temps de résidence est de l'ordre de 35 mn.

Les débits sont régulés parle pH, qui varie entre 6,7 et 7,1 pendant toute la durée de la réaction. Le produit de la réaction (produit R1) est récupéré en continu dans un autre réacteur, filtré et lavé par trois fois 12 litres d'eau. A l'issue de cette étape 2, on dispose du précurseur R2 contenant au moins 85 % en poids de phase rodérite.

Le précurseur est ensuite remis en suspension dans environ 250 cm$^3$ d'eau bipermutée, puis séché dans un atomiseur de type BUCHI (température de sortie = 145 °C, temps de séjour moyen t = 2 seconde). Le produit R3 obtenu contient environ 68 % d'oxydes.

Le précurseur R3 est ensuite calciné à 300 °C sous air (produit R4). La teneur en matières volatiles résiduelles est alors de 3 %.

La poudre R4 obtenue est mélangée avec 2 % en poids de graphite, pastillée en cylindre de 2,4 mm de diamètre et de 2 mm de hauteur, puis activé à 300 °C sous air. On obtient environ 133 g de catalyseur F.

### Exemple 7: Catalyseur G (comparatif)

On dissout 200,95 g de nitrate de cuivre trihydraté (0,83 at at.g Cu),384,55g de nitrate d'aluminium nonahydraté (1,03 at.g Al), 67,77 g de zinc hexahydraté (0,23 at.g Zn) dans 2 litres d'eau bipermutée, afin d'obtenir une solution (solution I) contenant 1,05 at.g de métaux par litre.

On dissout séparément 287,33 g de carbonate dissodique dans 4 litres d'eau. On obtient une solution II contenant 1,35 at.g de sodium par litre.

La réaction est menée dans un réacteur de 3 litres opérant en continu. Les deux solutions I et II sont injectées simultanément dans le réacteur contenant au préalable 2,8 litres d'eau bipermutée à une température de 50 à 60 °C. La température est maintenue entre 50 et 60 °C pendant toute la durée de la précipitation. Le temps de résidence est de l'ordre de 35 mn.

Les débits sont régulés par le pH, qui varie entre 6,7 et 7,1 pendant toute la durée de la réaction. Le produit de la réaction (produit P1) est récupéré en continu dans un autre réacteur, filtré et lavé par trois fois 12 litres d'eau. A l'issue de cette étape 2, on dispose du précurseur P2 contenant au moins 85 % en poids de phase prespinelle.

Le produit P2 est ensuite remis en suspension dans environ 500 cm$^3$ d'eau bipermutée et séché par atomisation à 130 °C pendant 2 secondes (produit P3 à 70 % d'oxydes), puis calciné à 300 °C sous air pour obtenir le produit P4, qui est broyé à l'échelle du micron dans un broyeur ALPINE.

La poudre P4 obtenue est mélangée avec 2 % en poids de graphite, pastillée en cylindre de 2,4 mm de diamètre et de 2 mm de hauteur, puis activé à 300 °C sous air. On obtient environ 136 g de catalyseur G.

### Exemple 8: Catalyseur H (comparatif)

On fait réagir une solution contenant 238,1 g de nitrate de cuivre(0,99 at.g Cu),238,14g de nitrate de zinc hexahydraté(0,86 at.g Zn)et 231,02g de nitrate d'aluminium nonahydraté (0,62at.g Al) dissous dans 2 litres d'eau bipermutée avec une solution (1,3 litre) de carbonate dissodique (339,6 g), à une température de 85 °C.

Le précipité est alors additionné de 50 g d'hydroxyde de sodium en pastilles, et le mélange porté à ébullition pendant 30 minutes.

Le précipité ainsi traité est alors filtré puis lavé quatre fois par 4 litres d'eau bipermutée afin d'enlever l'excès de sels solubles et plus particulièrement d'hydroxyde et de carbonate de sodium. Le précurseur ainsi obtenu est ensuite séché à l'étuve à 110 °C, puis calciné à 380 °C pendant 4 heures pour obtenir le catalyseur H, et enfin pastillé sous forme de pastilles de hauteur 2 mm et de 2,4 mm de diamètre puis activé à 380° C sous air.

### Exemple 9: Catalyseur I (comparatif)

On dissout 390 g d'aluminate de sodium dans 2 litres d'eau bipermutée et on y ajoute 1,1 litre d'une solution d'acide nitrique à 70 % poids. A cette solution, on ajoute ensuite 597 g de nitrate de zinc hexahydraté dissout dans 6 litres d'eau.

La solution résultante est chauffée à 85 °C et introduite dans un réacteur agité simultanément à une solution molaire de carbonate de sodium préalablement chauffée à 85 °C au moyen de pompes régulées par le pH. Le pH de la solution est maintenu à pH = 6,5 et la température à 65 °C. On obtient ainsi un précipité qui est filtré et lavé.

Une partie de ce précipité (1 390 g) est remise en suspension dans 3 litres d'eau et mélangée à une solution contenant 435 g de nitrate de cuivre trihydraté et 134 g de nitrate de zinc. A ce mélange, chauffé à 85 °C, on ajoute dans un réacteur agité une solution molaire de carbonate de sodium préalablement chauffé à 85 °C.

Le pH de la solution est maintenu à pH = 6,5 et la température à 65 °C en régulant le débit des pompes permettant l'alimentation en solutions de nitrate et de carbonate. Le mélange obtenu est mûri en chauffant à 85 °C e n un temps d'environ 10 minutes, puis en maintenant la température à 85 °C pendant 20 minutes supplémentaires sous une légère agitation. Le précipité est filtré, lavé afin d'obtenir une teneur en sodium inférieure à 0,1 % (exprimé en $Na_2O$), puis séché une nuit à 110 °C. Le produit ainsi obtenu est calciné pendant 6 heures à 300 °C, puis broyé à une granulométrie inférieure à 0,5 mm, et mélangé à 2 % en poids de graphite et pastillé en cylindre de 2,4 mm de diamètre et de 2mm de hauteur, puis activé à 300° C sous air.

### Exemple 10: Catalyseur J (comparatif)

On dissout 70,1 g de nitrate de cuivre trihydraté (0,29 at.g Cu), 33,8 g de nitrate d'aluminium nonahydraté (0,09 at. g Al), 35,7 g de nitrate de zinc hexahydraté (0,12 at.g Zn) dans 1 litre d'eau bipermutée, afin d'obtenir une solution (solution I) contenant 0,5 at.g de métaux par litre.

On dissout séparément 70 g de carbonate dissodique dans 0,7 litre d'eau. On obtient une solution II contenant 1,9 at.g de sodium par litre.

La réaction est menée dans un réacteur de 3 litres contenant 0,5 l d'eau bipermutée.

Les solutions I et II sont amenés au réacteur simultanément avec un débit de 0,5 litres par heure. La température est maintenue à 60 °C et le pH est maintenu constant et égal à pH = 7 par ajustement du débit de la solution de carbonate de sodium. Le précipité est ensuite mûri dans les eaux mères à 25 °C pendant une nuit, puis filtré et lavé par 2,5 litres d'eau. Le précipité lavé est ensuite séché à 75 °C puis calciné 1 nuit sous air à 350 °C. La teneur en matières volatiles résiduelles est alors de 2%.

La poudre obtenue par broyage en-dessous de 0,5 mm est mélangée avec 2 % en poids de graphite, pastillée en cylindre de 2,4 mm de diamètre et de 2 mm de hauteur, puis activée à 300 °C sous air. On obtient 37 g de catalyseur J.

### Exemple 11 : Catalyseur K (comparatif)

On dissout 30,3 g de nitrate de cuivre trihydraté (0,13 at.g Cu), 45,2 g de nitrate d'aluminium nonahydraté (0,12 at. g Al), 76,2 g de nitrate de zinc hexahydraté (0,26 at.g Zn) dans 1 litre d'eau bipermutée, afin d'obtenir une solution (solution I) contenant 0,5 at.g de métaux par litre.

On dissout séparément 69 g de carbonate dissodique dans 0,65 litre d'eau. On obtient une solution II contenant 2 at.g de sodium par litre.

La réaction est menée dans un réacteur de litres contenant 0,5 l d'eau bipermutée.

Les solutions I et II sont amenés au réacteur simultanément avec un débit de 0,5 litres par heure. La température est maintenue à 80 °C et le pH est maintenu constant et égal à pH = 7 par ajustement du débit de la solution de carbonate de sodium. Le précipité est ensuite mûri dans les eaux mères à 80 °C pendant une nuit, puis filtré et lavé par 2,5 litres d'eau à 80 °C. Le précipité lavé est ensuite séché à 75 °C puis calciné 1 nuit sous air à 350 °C. La teneur en matières volatiles résiduelles est alors de 3%.

La poudre obtenue par broyage en-dessous de 0,5 mm est mélangée avec 2 % en poids de graphite, pastillée en cylindre de 2,4 mm de diamètre et de 2 mm de hauteur, puis activée à 300 °C sous air. On obtient 37 g de catalyseur K.

Tous les catalyseurs décrits dans les exemples 1 à 11 sont testés en synthèse du méthanol en phase liquide dans un réacteur de 4 cm de diamètre et de 3 mètres de hauteur.

Les catalyseurs sont préalablement réduits in situ en phase gazeuse par un mélange d'hydrogène et d'azote contenant 6 % d'hydrogène dans l'azote, par paliers de températures successifs entre 160 °C et 240 °C à la pression atmosphérique.

Après réduction on injecte simultanément dans le réacteur le solvant (coupe paraffinique de $C_{12}$ à $C_{18}$) à raison de 270 litres par heure et le gaz de synthèse sous une pression de 6 MPa. Gaz et liquide circulent de haut en bas et les conditions opératoires sont les suivantes:

| Pression: | 6 MPa |
|---|---|
| Température: | 215 °C |
| Rapport $H_2/2CO + 3CO_2$ = | 1 |
| Vitesse volumique horaire: | 15 800 $h^{-1}$ |

Le tableau II indique la composition des catalyseurs, les proportions en chaque élément dans le catalyseur exprimées en pourcentage en poids de métal par rapport au poids total des métaux, ainsi que les résultats des tests en phase liquide (pour les exemples 1 à 11).

Les performances sont définies comme suit:

- Productivité massique en méthanol : c'est le nombre de grammes de méthanol obtenus par heure, ramenés au poids (en grammes) de catalyseur chargé (prod)
- Sélectivité en méthanol: c'est le rapport du nombre de moles de méthanol formées sur le nombre de moles de ($CO + CO_2$) disparues.

La sélectivité $S_M$ en méthanol s'exprime donc par :

$$S_M = 100 \times \frac{\text{nombre de moles de méthanol formées}}{\text{moles}(CO + CO_2)_{\text{entrée}} - \text{moles}(CO + CO_2)_{\text{sortie}}}$$

- Nombre de rotation : c'est le nombre de moles de méthanol obtenues par heure, ramenées à la surface métallique de cuivre accessible $S_{Cu}$ :

$$NR \ (\text{moles.m}^2 Cu^{-1}.h^{-1}) = \frac{\text{nombre de moles de méthanol formées par gramme de catalyseur}}{S_{Cu}(m^2/g. \ cat) \times \text{temp de réaction (h)}}$$

La surface métallique développée par le cuivre dans les catalyseurs de la présente invention et les catalyseurs des exemples comparatifs a été déterminée à partir de la dissociation de $N_2O$ à température ambiante selon la réaction :

$$2 \ Cu° + N_2O \rightarrow Cu_2O + N_2$$

Le catalyseur est préalablemant réduit in-situ sous hydrogène. La vitesse de montée en température est de 2°C/mm entre l'ambiante et 280° C. Après un palier d'une heure sous hydrogène, le catalyseur est désorbé sous hélium à 280° C pendant 1 heure, puis ramené à la température ambiante. La chimisorption de $N_2O$ est alors réalisée selon la technique chromatographique pulsée décrite par T.J. OSINGA et coll (J. Catal. 7,277,1967).

A la lecture du tableau II, on constate que les catalyseurs préparés selon l'invention (A à E) sont plus performants que les catalyseurs préparés selon l'art antérieur (F à K) : on obtient en effet, grâce aux catalyseurs A à E, une productivité massique et un nombre de rotation très supérieurs, tout en ayant une sélectivité en méthanol au fins aussi bonne, qu'avec les catalyseurs F à K.

## Exemple 12

Le catalyseur A est préalablement réduit in situ en phase gazeuse par un mélange d'hydrogène et d'azote contenant 6 % d'hydrogène dans l'azote, par paliers de température successifs entre 160° C et 240° C à la pression atmosphérique.

Après réduction on injecte le gaz de synthèse dans le réacteur sans une pression de 6 MPa.

Les conditions opératoires sont les suivantes :

| | |
|---|---|
| Pression : | 6 MPa |
| Température : | 215° C |
| Rapport $H_2/2CO+3CO_2$ = | 1 |
| Vitesse volumétrique horaire : | 15800 $h^{-1}$ |

Les performances sont indiquées au tableau II.

On remarque que même lors d'un procédé de synthèse du méthanol en phase gazeuse, le catalyseur A préparé selon l'invention présente de très bons résultats.

## Exemple 13

Dans un réacteur tubulaire, 10 g de catalyseur A sont réduits quelques heures à pression atmosphérique par 5 % d'hydrogène dans l'azote entre 140 et 260°C. Après réduction, on procède à la réaction de décomposition du méthanol. Le mélange eau-méthanol admis au réacteur contient 2 moles d'eau parmole de méthanol.

Pour une température de 290°C, une pression de 3 MPa et une vitesse spatiale liquide égale à 3 $h^{-1}$, plus de 99 % du méthanol sont convertis. Le rendement en hydrogène, exprimé par la relation

$$R = 100 \times \frac{H}{3CH_3OH} (mol)$$

est supérieur à 99 %.

Au bout de 500 heures d'opération, les performances demeurent inchangées.

Le catalyseur A préparé selon l'invention présente donc de très bons résultats en décomposition du méthanol pour la production d'hydrogène.

**TABLEAU II**

| Exemple | Catalyseurs Réf. | Catalyseurs Formule | Cu% | Al% | Zn% | $\frac{Cu+Zn}{Al}$ R | $\frac{Cu+Zn}{Al}$ P | $\frac{Zn}{Al}$ R | $\frac{Zn}{Al}$ P | SCu | Prod. | SM(%) | NR |
|---|---|---|---|---|---|---|---|---|---|---|---|---|---|
| 1 | A | $Cu_{1,46} Al_{0,94} Zn_{1,24}$ | 46,6 | 12,7 | 40,7 | 12,7 | 1,5 | 4,0 | 0,9 | 33 | 1,45 | 99,5 | 1,40 |
| 2 | B | $Cu_{0,82} Al_{1,18} Zn_{0,85}$ | 37,3 | 22,8 | 39,8 | 4,2 | 0,3 | 2,1 | 0,2 | 27 | 1,18 | 99,6 | 1,37 |
| 3 | C | $Cu_{0,82} Al_{1,18} Zn_{0,85}$ | 37,3 | 22,8 | 39,8 | 4,2 | 0,3 | 2,1 | 0,2 | 23 | 1,02 | 99,5 | 1,38 |
| 4 | D | $Cu_{1,58} Al_{1,24} Zn_{1,08}$ | 49,1 | 16,4 | 34,5 | 7,5 | 1,0 | 4,0 | 0,2 | 32 | 1,34 | 99,4 | 1,31 |
| 5 | E | $Cu_{1,58} Al_{1,24} Zn_{1,08}$ | 49,1 | 16,4 | 34,5 | 7,5 | 1,0 | 4,0 | 0,2 | 35 | 1,55 | 99,7 | 1,38 |
| 6 | F | $Cu_{0,72} Al_{0,34} Zn_{0,72}$ | 44,8 | 9,0 | 46,2 | 4,2 | - | 2,1 | - | 20 | 0,64 | 99,3 | 1,00 |
| 7 | G | $Cu_{0,83} Al_{1,03} Zn_{0,23}$ | 55,2 | 29,1 | 15,7 | - | 1,0 | - | 0,2 | 27 | 0,82 | 98,5 | 0,95 |
| 8 | H | $Cu_{0,99} Al_{0,62} Zn_{0,86}$ | 46,3 | 12,3 | 41,4 | GLOBAL 3,0 | | GLOBAL 1,4 | | 20 | 0,55 | 99,2 | 0,86 |
| 9 | I | $Cu_{6,0} Al_{2,33} Zn_{1,67}$ | 68,9 | 11,4 | 19,7 | 3,3 | | 0,7 | | 27 | 0,95 | 98,8 | 1,10 |
| 10 | J | $Cu_{0,29} Al_{0,09} Zn_{0,12}$ | 64,2 | 8,5 | 27,3 | 4,6 | | 1,3 | | 24 | 0,84 | 99,2 | 1,09 |
| 11 | K | $Cu_{0,13} Al_{0,12} Zn_{0,26}$ | 28,9 | 11,4 | 59,6 | 3,2 | | 2,2 | | 15 | 0,50 | 99,4 | 1,04 |
| 12 | A | $Cu_{1,46} Al_{0,94} Zn_{1,24}$ | 46,6 | 12,7 | 40,7 | 12,7 | 1,5 | 4,0 | 0,9 | 33 | 1,31 | 99,6 | 1,24 |

( R = phase rodérite , P = phase prespinelle )

## Revendications

1. Procédé de préparation d'un précurseur de catalyseur contenant du cuivre, de l'aluminium et du zinc, au moins 50% en poids dudit précurseur étant formé par un mélange d'une phase appelée rodérite constituée de cuivre, d'aluminium et de zinc et ayant un rapport atomique $\frac{Cu+Zn}{Al}$ compris entre 3,5 et 16 et un rapport atomique $\frac{Zn}{Al}$

compris entre 1,6 et 6 et d'une phase appelée prespinelle microcristallisée constituée de cuivre, d'aluminium et de zinc et ayant un rapport atomique $\frac{Cu+Zn}{Al}$ compris entre 0,20 et 2,10 et un rapport atomique $\frac{Zn}{Al}$ compris entre 0, 10 et 1,50, caractérisé en ce que ledit précurseur résulte du mélange de deux précurseurs ternaires comprenant chacun du cuivre, de l'aluminium et du zinc, l'un desdits précurseurs ternaires contenant au moins 50 % en poids de phase rodérite et l'autre desdits précurseurs ternaires contenant au moins 50 % en poids de phase prespinelle microcristallisée, ledit mélange ayant lieu entre, d'une part, l'un des précurseurs ternaires obtenus à l'issue de l'une des étapes 1) à 4) d'un procédé P comprenant les étapes suivantes:

1) réaction de coprécipitation au sein d'un réacteur contenant préalablement de l'eau entre une solution aqueuse d'au moins un composé d'un constituant choisi dans le groupe formé par les métaux alcalins et l'ion ammonium, ledit composé étant choisi dans le groupe formé par les carbonates, les hydrogénocarbonates et les hydroxydes, et une solution aqueuse contenant, sous forme de composés choisis dans le groupe formé par les sels solubles et les complexes solubles, du cuivre, de l'aluminium et du zinc dans des proportions telles que le rapport atomique $\frac{Cu+Zn}{Al}$ est compris entre 3,5 et 16 et le rapport atomique $\frac{Zn}{Al}$ est compris entre 1,6 et 6, ladite réaction ayant lieu à un pH compris entre 6,3 et 7,3, à une température comprise entre 70 et 90 °C, avec un temps de résidence compris entre 0,1 et 60 minutes,
2) filtration, puis lavage à l'eau du produit obtenu à l'issue de l'étape 1),
3) séchage du produit obtenu à l'issue de l'étape 2), entre 50 et 200 °C,
4) calcination du produit obtenu à l'issue de l'étape 3), entre 250 et 500 °C,

et, d'autre part, l'un des précurseurs tel obtenus à l'issue de l'une des étapes 1) à 4) d'un procédé P' comprenant les étapes suivantes:

1) réaction de coprécipitation au sein d'un réacteur contenant préalablement de l'eau entre une solution aqueuse d'au moins un composé d'un constituant choisi dans le groupe formé par les métaux alcalins et l'ionammonium, ledit composé étant choisi dans le groupe formé par les carbonates, les hydrogénocarbonates et les hydroxydes, et une solution aqueuse contenant, sous forme de composés choisis dans le groupe formé par les sels solubles et les complexes solubles, du cuivre, de l'aluminium et du zinc dans des proportions telles que le rapport atomique $\frac{Cu+Zn}{Al}$ est compris entre 0,20 et 2,10 et le rapport atomique $\frac{Zn}{Al}$ est compris entre 0,10 et 1,50, ladite réaction ayant lieu à un pH compris entre 6,3 et 7,3, à une température comprise entre 45 et 65 °C, avec un temps de résidence compris entre 0,1 et 60 minutes,
2) filtration, puis lavage à l'eau du produit obtenu à l'issue de l'étape 1),
3) séchage du produit obtenu à l'issue de l'étape 2), entre 50 et 200 °C,
4) calcination du produit obtenu à l'issue de l'étape 3), entre 250 et 500 °C,

ledit mélange étant suivi :

- d'une filtration et d'un lavage à l'eau, puis d'un séchage entre 50 et 200 °C, puis d'une calcination entre 250 et 500 °C, si au moins l'un des deux précurseurs ternaires qui ont été mélangés a été préalablement obtenu à l'issue de l'étape 1) de l'un des procédés P et P';
- d'un séchage entre 50 et 200 °C, puis d'une calcination entre 250 et 500 °C, si l'un des deux précurseurs ternaires qui ont été mélangés a été préalablement obtenu à l'issue de l'étape 2) de l'un des procédés P et P', l'autre des deux précurseurs ternaires qui ont été mélangés ayant été obtenu à l'issue de l'une des étapes 2) à 4) de l'un des procédés P et P';
- d'une calcination entre 250 et 500 °C, si les deux précurseurs ternaires qui ont été mélangés ont été préalablement obtenus à l'issue de l'une des étapes 3) et 4) de l'un des procédés P et P'.

2. Procédé selon la revendication 1 caractérisé en ce qu'il comprend les étapes suivantes:

a) réaction de coprécipitation selon l'étape 1) du procédé P de la revendication 1,
b) filtration puis lavage à l'eau du produit obtenu à l'issue de l'étape a),
c) réaction de coprécipitation selon l'étape 1) du procédé P' de la revendication 1,
d) filtration puis lavage à l'eau du produit obtenu à l'issue de l'étape c),
e) mélange du produit obtenu à l'issue de l'étape b) avec le produit obtenu à l'issue de l'étape d),
f) séchage du produit obtenu à l'issue de l'étape e), entre 50 et 200 °C,
g) calcination du produit obtenu à l'issue de l'étape f), entre 250 et 500 °C.

3. Procédé selon la revendication 1 caractérisé en ce qu'il comprend les étapes suivantes:

EP 0 395 471 B2

a) réaction de coprécipitation selon l'étape 1) du procédé P de la revendication 1,

b) filtration puis lavage à l'eau du produit obtenu à l'issue de l'étape a),

c) séchage du produit obtenu à l'issue de l'étape b), entre 50 et 200 °C,

d) réaction de coprécipitation selon l'étape 1) du procédé P' de la revendication 1,

e) filtration puis lavage à l'eau du produit obtenu à l'issue de l'étape d),

f) séchage du produit obtenu à l'issue de l'étape e), entre 50 et 200 °C,

g) mélange du produit obtenu à l'issue de l'étape c) avec le produit obtenu à l'issue de l'étape f),

h) calcination du produit obtenu à l'issue de l'étape g), entre 250 et 500 °C.

**4.** Procédé selon la revendication 1 caractérisé en ce qu'il comprend les étapes suivantes:

a) réaction de coprécipitation selon l'étape 1) du procédé P de la revendication 1,

b) filtration puis lavage à l'eau du produit obtenu à l'issue de l'étape a),

c) séchage du produit obtenu à l'issue de l'étape b), entre 50 et 200 °C,

d) calcination du produit obtenu à l'issue de l'étape c), entre 250 et 500 °C,

e) réaction de coprécipitation selon l'étape 1) du procédé P' de la revendication 1,

f) filtration puis lavage à l'eau du produit obtenu à l'issue de l'étape e),

g) séchage du produit obtenu à l'issue de l'étape f), entre 50 et 200 °C,

h) calcination du produit obtenu à l'issue de l'étape g), entre 250 et 500 °C,

i) mélange du produit obtenu à l'issue de l'étape d) avec le produit obtenu à l'issue de l'étape h),

j) calcination du produit obtenu a l'issue de l'étape i), entre 250 et 500 °C.

**5.** Procédé selon la revendication 1 caractérisé en ce qu'il comprend les étapes suivantes:

a) réaction de coprécipitation selon l'étape 1) du procédé P de la revendication 1,

b) filtration puis lavage à l'eau du produit obtenu à l'issue de l'étape a),

c) séchage du produit obtenu à l'issue de l'étape b), entre 50 et 200 °C,

d) réaction de coprécipitation selon l'étape 1) du procédé P' de la revendication 1,

e) filtration puis lavage à l'eau du produit obtenu à l'issue de l'étape d),

f) mélange du produit obtenu à l'issue de l'étape c) avec le produit obtenu à l'issue de l'étape e),

g) séchage du produit obtenu à l'issue de l'étape h),entre 50 et 200 °C,

h) calcination du produit obtenu à l'issue de l'étape g), entre 250 et 500 °C.

**6.** Procédé selon la revendication 1 caractérisé en ce qu'il comprend les étapes suivantes:

a) réaction de coprécipitation selon l'étape 1) du procédé P de la revendication 1,

b) filtration puis lavage à l'eau du produit obtenu à l'issue de l'étape a),

c) séchage du produit obtenu à l'issue de l'étape b), entre 50 et 200 °C,

d) calcination du produit obtenu à l'issue de l'étape c), entre 250 et 500 °C,

e) réaction de coprécipitation selon l'étape 1) du procédé P' de la revendication 1,

f) filtration puis lavage à l'eau du produit obtenu à l'issue de l'étape e),

g) mélange du produit obtenu à l'issue de l'étape d) avec le produit obtenu à l'issue de l'étape f).

h) séchage du produit obtenu à l'issue de l'étape g), entre 50 et 200 °C,

i) calcination du produit obtenu à l'issue de l'étape h), entre 250 et 500 °C.

**7.** Procédé selon la revendication 1 caractérisé en ce qu'il comprend les étapes suivantes:

a) réaction de coprécipitation selon l'étape 1) du procédé P' de la revendication 1,

b) filtration puis lavage à l'eau du produit obtenu à l'issue de l'étape a),

c) séchage du produit obtenu à l'issue de l'étape b), entre 50 et 200 °C,

d) réaction de coprécipitation selon l'étape 1) du procédé P de la revendication 1,

e) filtration puis lavage à l'eau du produit obtenu à l'issue de l'étape d),

f) mélange du produit obtenu à l'issue de l'étape c) avec le produit obtenu à l'issue de l'étape e),

g) séchage du produit obtenu à l'issue de l'étape h), entre 50 et 200 °C,

h) calcination du produit obtenu à l'issue de l'étape g), entre 250 et 500 °C.

**8.** Procédé selon la revendication 1 caractérisé en ce qu'il comprend les étapes suivantes:

a) réaction de coprécipitation selon l'étape 1) du procédé P' de la revendication 1,

b) filtration puis lavage à l'eau du produit obtenu à l'issue de l'étape a),

c) séchage du produit obtenu à l'issue de l'étape b), entre 50 et 200 °C,

d) calcination du produit obtenu à l'issue de l'étape c), entre 250 et 500 °C,

e) réaction de coprécipitation selon l'étape 1) du procédé P de la revendication 1,

f) filtration puis lavage à l'eau du produit obtenu à l'issue de l'étape e),

g) mélange du produit obtenu à l'issue de l'étape d) avec le produit obtenu à l'issue de l'étape f),

h) séchage du produit obtenu à l'issue de l'étape g), entre 50 et 200 °C,

i) calcination du produit obtenu à l'issue de l'étape h), entre 250 et 500 °C.

9. Procédé selon la revendication 1 caractérisé en ce qu'il comprend les étapes suivantes:

a) réaction de coprécipitation selon l'étape 1) du procédé P de la revendication 1,

b) filtration puis lavage à l'eau du produit obtenu à l'issue de l'étape a),

c) séchage du produit obtenu à l'issue de l'étape b), entre 50 et 200 °C,

d) calcination du produit obtenu à l'issue de l'étape c), entre 250 et 500 °C,

e) réaction de coprécipitation selon l'étape 1) du procédé P' de la revendication 1,

f) filtration puis lavage à l'eau du produit obtenu à l'issue de l'étape e),

g) séchage du produit obtenu à l'issue de l'étape f), entre 50 et 200 °C,

h) mélange du produit obtenu à l'issue de l'étape d) avec le produit obtenu à l'issue de l'étape g),

i) calcination du produit obtenu à l'issue de l'étape h), entre 250 et 500 °C.

10. Procédé selon la revendication 1 caractérisé en ce qu'il comprend les étapes suivantes :

a) fraction de coprécipitation selon l'étape 1) du procédé P' de la revendication 1,

b) filtration puis lavage à l'eau du produit obtenu à l'issue de l'étape a),

c) séchage du produit obtenu à l'issue de l'étape b), entre 50 et 200°C

d) calcination du produit obtenu à l'issue de l'étape c), entre 250 et 500° C.

e) réaction de coprécipitation selon l'étape 1) du procédé P de la revendication 1,

f) filtration puis lavage à l'eau du produit obtenu à l'issue de l'étape e),

g) séchage du produit obtenu à l'issue de l'étape g), entre 50 et 200°C

h) mélange du produit obtenu à l'issue de l'étape d), avec ce produit obtenu à l'issue de l'étape g),

i) calcination du produit obtenu à l'issue de l'étape h), entre 250 et 500° C.

11. Procédé selon la revendication 1 caractérisé en ce qu'il comprend les étapes suivantes :

a) réaction de coprécipitation selon l'étape 1) du procédé P de la revendication 1,

b) réaction de coprécipitation selon l'étape 1) du procédé P' de la revendication 1,

c) mélange du produit obtenu à l'issue de l'étape a) avec le produit obtenu à l'issue de l'étape b),

d) filtration puis lavage à l'eau du produit obtenu à l'issue de l'étape c),

e) séchage du produit obtenu à l'issue de l'étape d), entre 50 et 200°C,

f) calcination du produit obtenu à l'issue de l'étape e), entre 250 et 500° C.

12. Procédé de préparation d'un précurseur de catalyseur selon l'une des revendications 1 à 11, caractérisé en ce que :

le mélange a lieu entre d'une part le précurseur obtenu à l'issue de l'étape 2) du procédé P, ledit précurseur étant mis en suspension dans l'eau, et d'autre part le précurseur ternaire obtenu à l'issue de l'étape 2 du procédé P', l'étape 1) dudit procédé P' ayant lieu dans la suspension du précurseur ternaire du procédé P, le mélange obtenu étant filtré puis lavé à l'eau, séché entre 50 et 200°C puis calciné entre 250 et 500° C.

13. Procédé de préparation d'un précurseur de catalyseur selon l'une des revendications 1 à 11, caractérisé en ce que :

le mélange a lieu entre d'une part le précurseur ternaire obtenu à l'issue de l'étape 2) du procédé P', ledit précurseur étant mis en suspension dans l'eau, et d'autre part le précurseur ternaire obtenu à l'issue de l'étape 2) du procédé P, l'étape 1) du procédé P ayant lieu dans la suspension du précurseur ternaire du procédé P', le mélange obtenu étant filtré puis lavé à l'eau, séché entre 50 et 200° C puis calciné entre 250 et 500°C.

14. Procédé selon l'une des revendications 1, 6, 8 à 10 comportant deux étapes de calcination, dans lequel la première étape de calcination est immédiatement suivie d'un broyage du produit obtenu de ladite première étape de calcination afin d'obtenir un produit sous forme de poudre de granulométrie inférieure à $2.10^{-6}$m.

**15.** Procédé selon l'une des revendications 1 et 4 comportant trois étapes de calcination, dans lequel au moins l'une des deux premières étapes de calcination est immédiatement suivie d'un broyage du produit obtenu à l'issue de ladite une des deux premières étapes de calcination afin d'obtenir un produit sous forme de poudre de granulométrie inférieure à $2.10^{-6}$m.

**16.** Procédé selon l'une des revendications 1 à 15 dans lequel au moins une étape de séchage est immédiatement suivie d'un broyage du produit obtenu à l'issue de ladite étape de séchage afin d'obtenir un produit sous forme de poudre de granulométrie inférieure à $2.10^{-6}$m.

**17.** Procédé selon l'une des revendications 1 à 15 dans lequel chaque étape de séchage est immédiatement suivie d'un broyage du produit obtenu à l'issue de ladite étape de séchage afin d'obtenir un produit sous forme de poudre de granulométrie inférieure à $2.10^{-6}$m.

**18.** Procédé selon l'une des revendications 1 à 17 de préparation d'un précurseur de catalyseur contenant du cuivre, de l'aluminium et du zinc, au moins 65 % en poids dudit précurseur étant formé par un mélange de phase rodérite et de phase prespinelle, ledit précurseur résultant du mélange de deux précurseurs ternaires comprenant chacun du cuivre, de l'aluminium et du zinc, l'un desdits précurseurs ternaires contenant au moins 65 % en poids de phase rodérite et l'autre desdits précurseurs ternaires contenant au moins 65 % en poids de phase prespinelle.

**19.** Procédé selon l'une des revendications 1 à 17 de préparation d'un précurseur de catalyseur contenant du cuivre, de l'aluminium et du zinc, au moins 85 % en poids dudit précurseur étant formé par un mélange de phase rodérite et de phase prespinelle, ledit précurseur résultant du mélange de deux précurseurs ternaires comprenant chacun du cuivre, de l'aluminium et du zinc, l'un desdits précurseurs ternaires contenant au moins 85 % en poids de phase rodérite et l'autre desdits précurseurs ternaires contenant au moins 85 % en poids de phase prespinelle.

**20.** Procédé selon l'une des revendications 1 à 19 de préparation d'un précurseur hydroxycarbonaté de catalyseur dans lequel ledit mélange s'effectue entre deux précurseurs ternaires hydroxycarbonatés.

**21.** Procédé de prépatation d'un catalyseur caractérisé en ce qu'il comprend les étapes suivantes :

1) broyage du précurseur préparé selon l'une des revendications 1 à 20 afin d'obtenir un produit sous forme de poudre de granulométrie inférieure à 1mm.
2) mise en forme du produit obtenu à l'issue de l'étape (1), incluant éventuellement un mélange produit à raison de 0,5 - 5% de son poids avec au moins un composé choisi dans le groupe formé par le graphite, l'acide stéarique, les stéarates.
3) calcination du produit obtenu à l'issue de l'étape 2), entre 250 et 500° C.

**22.** Précurseur contenant en poids 15 à 80 % de cuivre, 4 à 50 % d'aluminium, et 10 à 70 % de zinc rapportées au poids total des métaux présents dans ledit précurseur, une partie de ces éléments se présentent en phase rodérite, caractérisé en ce que l'autre partie de ces éléments se trouve en phase préspinelle qui présente un rapport atomique (Cu+ Zn)/Al compris entre 0,20 et 2,10 et un rapport atomique Zn/Al compris entre 0,10 et 1,50 et en ce qu'elle présente, en spectre infra-rouge, une vibration ($CO_3$2-) conduisant à une bande longue dont le maximun est situé à 1500 cm$^{-1}$ et une bande fine à 1390 cm$^{-1}$, lesdites phases représentent plus de 50 % du poids du précurseur.

**23.** Catalyseur contenant en poids 15 à 80 % de cuivre, 4 à 50 % d'aluminium et 10 à 70 % de zinc rapportées au poids total des métaux présents dans ledit catalyseur, une partie de ces éléments se présentent en phase rodérite, caractérisé en ce que l'autre partie de ces éléments se trouve en phase préspinelle qui présente un rapport atomique (Cu+ Zn)/Al compris entre 0,20 et 2,10 et un rapport atomique Zn/Al compris entre 0,10 et 1,50 et en ce qu'elle présente, en spectre infra-rouge, une vibration ($CO_3$2-) conduisant à une bande longue dont le maximun est situé à 1500 cm$^{-1}$ et une bande fine à 1390 cm$^{-1}$.

**24.** Utilisation d'un catalyseur selon la revendication 23 ou prépare selon la revendication 21 dans un procédé mettant en jeu une réaction équilibrée des oxydes de carbone CO et $CO_2$ avec l'hydrogène.

**25.** Utilisation d'un catalyseur selon la revendication 23 ou préparé selon la revendication 21 dans un procédé de production de méthanol à partir d'oxydes de carbone CO et $CO_2$ et d'hydrogène.

**26.** Utilisation d'un catalyseur selon la revendication 23 ou préparé selon la revendication 21 dans un procédé de décomposition d'au moins un alcool primaire de $C_1$ à $C_5$ en oxyde de carbone CO et $CO_2$ et hydrogène.

**Claims**

1. A process for preparing a precursor of catalyst containing copper, aluminum and zinc, at least 50% by weight of said precursor being made up of the mixing of a phase called rodorite consisting of copper, aluminum and zinc and showing an atomic ratio (Cu + Zn)/Al ranging from 3.5 to 16 and an atomic ration Zn/Al ranging from 1.6 to 6, and of a phase called microcrystallized prespinel consisting of copper, aluminum and zinc and showing an atomic ratio (Cu + Zn)/Al ranging from 0.20 to 2.10 and an atomic ratio Zn/Al ranging from 0.10 to 1.50, characterized in that said precursor results from the mixing of two ternary precursors each comprising copper, aluminum and zinc, one of said ternary precursors containing at least 50% by weight of roderite phase and the other one of said ternary precursors containing at least 50% by weight of microcrystallized prespinal phase, said mixing taking place between, on one hand, one of the ternary precursors obtained at the end of one of stages 1) to 4) of a process P comprising the following stages:

> 1) co-precipitation reaction within a reactor previously containing water between an aqueous solution of at least one compound of a constituent selected from the group consisting of the alkali metals and the ammonium ion, said compound being selected from the group consisting of the carbonates, the hydrogenocarbonates and the hydroxydes, and an aqueous solution containing, in the form of compounds selected from the group consisting of the soluble salts and the soluble complexes, copper, aluminum and zinc in such proportions that the atomic ratio (Cu + Zn)/Al ranges from 3.5 to 16 and the atomic ratio Zn/Al ranges from 1.6 to 6, said reaction taking place at a pH value ranging from 6.3 to 7.3, at a temperature ranging from 70 to 90°C, with a residence time ranging from 0.1 to 60 minutes,
> 2) filtration, then water washing of the product obtained at the end of stage 1),
> 3) drying of the product obtained at the end of stage 2), between 50 and 200°C,
> 4) calcination of the product obtained at the end of stage 3), between 250 and 500°C,

and, on the other hand, one of the ternary precursors obtained at the end of one of stages 1) to 4) of a process P' comprising the following stages:

> 1) co-precipitation reaction within a reactor previously containing water between an aqueous solution of at least one compound of a constituent selected from the group consisting of the alkali metals and the ammonium ion, said compound being selected from the group consisting of the carbonates, the hydrogenocarbonates and the hydroxydes, and an aqueous solution containing, in the form of compounds selected from the group consisting of the soluble salts and the soluble complexes, copper, aluminum and zinc in such proportions that the atomic ratio (Cu + Zn)/Al ranges from 0.20 to 2.10 and the atomic ratio Zn/Al ranges from 0.10 to 1.50, said reaction taking place at a pH value ranging from 6.3 to 7.3, at a temperature ranging from 45 to 65°C with a residence time ranging from 0.1 to 60 minutes,
> 2) filtration, then water washing of the product obtained at the end of stage 1),
> 3) drying of the product obtained at the end of stage 2), between 50 and 200°C,
> 4) calcination of the product obtained at the end of stage 3), between 250 and 500°C.

said mixing being followed by:

- filtering and water washing, then drying between 50 and 200°C, and afterwards calcining between 250 and 500°C, if at least one of the two ternary precursors which have been mixed together has been previously obtained at the end of stage i) of one of the processes P and P';
- drying between 50 and 200°C, then calcining between 250 and 500°C, if one of the ternary precursors which have been mixed together has been previously obtained at the end of stage 2) of any one of processes P and P', the other one of the two ternary precursors which have been mixed together being obtained at the end of one of stages 2) to 4) of one of the processes P and P';
- calcining between 250 and 500°C, if the two ternary precursors which have been mixed together have been previously obtained at the end of one of stages 3) and 4) of one of the processes P and P'.

2. A process according to claim 1, characterized in that it comprises the following stages:

> a) co-precipitation reaction according to stage 1) of process P of claim 1,
> b) filtration, then water washing of the product obtained at the end of stage a),
> c) co-precipitation reaction according to stage 1) of process P' of claim 1,
> d) filtration, then water washing of the product obtained at the end of stage c),
> e) mixing of the product obtained at the end of stage b) with the product obtained at the end of stage d),

f) drying of the product obtained at the end of stage c), between 50 and 200°C,

g) calcination of the product obtained at the end of stage f), between 250 and 500°C.

3. A process according to claim 1, characterized in that it comprises the following stages:

a) co-precipitation reaction according to stage 1) of process P of claim 1,

b) filtration, then water washing of the product obtained at the end of stage a),

c) drying of the product obtained at the end of stage b), between 50 and 200°C,

d) co-precipitation reaction according to stage 1) of process P' of claim 1,

e) filtration, then water washing of the product obtained at the end of stage d),

f) drying of the product obtained at the end of stage e), between 50 and 200°C,

g) mixing of the product obtained at the end of stage c) with the product obtained at the end of stage f),

h) calcination of the product obtained at the end of stage g), between 250 and 500°C.

4. A process according to claim 1, characterized in that it comprises the following stages:

a) co-precipitation reaction according to stage 1) of process P of claim 1,

b) filtration, then water washing of the product obtained at the end of stage a),

c) drying of the product obtained at the end of stage b), between 50 and 200°C,

d) calcination of the product obtained at the end of stage c), between 250 and 500°C,

e) co-precipitation reaction according to stage 1) of process P' of claim 1,

f) filtration, then water washing of the product obtained at the end of stage e),

g) drying of the product obtained at the end of stage f), between 50 and 200°C,

h) calcination of the product obtained at the end of stage g), between 250 and 500°C,

i) mixing of the product obtained at the end of stage d) with the product obtained at the end of stage h),

j) calcination of the product obtained at the end of stage i), between 250 and 500°C.

5. A process according to claim 1, characterized in that it comprises the following stages:

a) co-precipitation reaction according to stage 1) of process P of claim 1,

b) filtration, then water washing of the product obtained at the end of stage a),

c) drying of the product obtained at the end of stage b), between 50 and 200°C,

d) co-precipitation reaction according to stage 1) of process P' of claim 1,

e) filtration, then water washing of the product obtained at the end of stage d),

f) mixing of the product obtained at the end of stage c) with the product obtained at the end of stage e),

g) drying of the product obtained at the end of stage f), between 50 and 200°C,

h) calcination of the product obtained at the end of stage g), between 250 and 500°C.

6. A process according to claim 1, characterized in that it comprises the following stages:

a) co-precipitation reaction according to stage 1) of process P of claim 1,

b) filtration, then water washing of the product obtained at the end of stage a),

c) drying of the product obtained at the end of stage b), between 50 and 200°C,

d) calcination of the product obtained at the end of stage c), between 250 and 500°C,

e) co-precipitation reaction according to stage 1) of process P' of claim 1,

f) filtration, then water washing of the product obtained at the end of stage e),

g) mixing of the product obtained at the end of stage d) with the product obtained at the end of stage f),

h) drying of the product obtained at the end of stage g), between 50 and 200°C,

i) calcination of the product obtained at the end of stage h), between 250 and 500°C.

7. A process according to claim 1, characterized in that it comprises two following stages:

a) co-precipitation reaction according to stage 1) of process P' of claim 1,

b) filtration, then water washing of the product obtained at the end of stage a),

c) drying of the product obtained at the end of stage b), between 50 and 200°C,

d) co-precipitation reaction according to stage 1) of process P of claim 1,

e) filtration, then water washing of the product obtained at the end of stage d),

f) mixing of the product obtained at the end of stage c) with the product obtained at the end of stage e),

g) drying of the product obtained at the end of stage f), between 50 and 200°C,

h) calcination of the product obtained at the end of stage g), between 250 and 500°C.

8. A process according to claim 1, characterized in that it comprises the following stages:

   a) co-precipitation reaction according to stage 1) of process P' of claim 1,
   b) filtration, then water washing of the product obtained at the end of stage a),
   c) drying of the product obtained at the end of stage b), between 50 and 200°C.
   d) calcination of the product obtained at the end of stage c), between 250 and 500°C,
   e) co-precipitation reaction according to stage 1) of process P of claim 1,
   f) filtration, then water washing of the product obtained at the end of stage e),
   g) mixing of the product obtained at the end of stage d), with the product obtained at the end of stage f),
   h) drying of the product obtained at the end of stage g), between 50 and 200°C,
   i) calcination of the product obtained at the end of stage h), between 250 and 500°C.

9. A process according to claim 1, characterized in that it comprises the following stages:

   a) co-precipitation reaction according to stage 1) of process P of claim 1,
   b) filtration, then water washing of the product obtained at the end of stage a),
   c) drying of the product obtained at the end of stage b), between 50 and 200°C,
   d) calcination of the product obtained at the end of stage c), between 250 and 500°C,
   e) co-precipitation reaction according to stage 1) of process P' of claim 1,
   f) filtration, then water washing of the product obtained at the end of stage e),
   g) drying of the product obtained at the end of stage f), between 50 and 200°C,
   h) mixing of the product obtained at the end of stage d) with the product obtained at the end of stage g),
   i) calcination of the product obtained at the end of stage h), between 250 and 500°C.

10. A process according to claim 1, characterized in that it comprises the following stages:

   a) co-precipitation reaction according to stage 1) of process P' of claim 1,
   b) filtration, then water washing of the product obtained at the end of stage a),
   c) drying of the product obtained at the end of stage b), between 50 and 200°C,
   d) calcination of the product obtained at the end of stage c), between 250 and 500°C,
   e) co-precipitation reaction according to stage 1) of process P of claim 1,
   f) filtration, then water washing of the product obtained at the end of stage e),
   g) drying of the product obtained at the end of stage f), between 50 and 200°C,
   h) mixing of the product obtained at the end of stage d) with the product obtained at the end of stage g)
   i) calcination of the product obtained at the end of stage h), between 250 and 500°C.

11. A process according to claim 1, characterized in that it comprises the following stages:

   a) co-precipitation reaction according to stage 1) of process P of claim 1,
   b) co-precipitation reaction according to stage 1) of process P' of claim 1,
   c) mixing of the product obtained at the end of stage a) with the product obtained at the end of stage b),
   d) filtration, then water washing of the product obtained at the end of stage c),
   e) drying of the product obtained at the end of stage d), between 50 and 200°C,
   f) calcination of the product obtained at the end of stage e), between 250 and 500°C.

12. A process for the preparation of a precursor of catalyst according to one of claims 1 to 11, characterized in that: the mixture takes place of the precursor obtained at the end of stage 2) in process P, on the one hand, said precursor being placed in suspension in water, and of the ternary precursor obtained at the end of stage 2 of process P', on the other hand, stage 1) of said process P' taking place in the suspension of the ternary precursor of the process P, the mixture obtained then being filtered and then water washed, dried between 50 and 200°C and then calcined between 250 and 500°C.

13. A process for the preparation of a precursor of catalyst according to one of claims 1 to 11, characterized in that: the mixture takes place of the ternary precursor, on the one hand, obtained at the end of stage 2) of the process P', said precursor being placed in suspension in water, and, on the other hand, of the ternary precursor obtained at the end of stage 2) of the process P, stage 1) of the process P taking place in the suspension of the ternary precursor of the process P', the mixture obtained being filtered, then water washed, dried between 50 and 200°C and then calcined between 250 and 500°C.

14. A process according to one of claims 1, 6, 8 to 10 comprising two calcination stages, wherein the first calcination stage is immediately followed by the grinding of the product obtained at the end of said first calcination stage in order to obtain a product in the form of a powder with a grain size smaller than 2.10-6m.

15. A process according to one of claims 1 and 4 comprising three calcination stages, wherein at least one of the first two calcination stages is immediately followed by the grinding of the product obtained at the end of said one of the first two calcination stages in order to obtain a product in the form of a powder with a graine size smaller than $2.10^{-6}$m.

16. A process according to one of claims 1 to 15 wherein at least one drying stage is immediately followed by the grinding of the product obtained at the end of said drying stage in order to obtain a product in the form of a powder with a grain size smaller than $2.10^{-6}$m.

17. A process according to one of claims 1 to 15 wherein each drying stage is immediately followed by the grinding of the product obtained at the end of said drying stage in order to obtain a product in the form of a powder with a grain size smaller than $2.10^{-6}$m.

18. A process according to one of claims 1 to 17 for preparing a precursor of catalyst containing copper, aluminum and zinc, at least 65% by weight of said precursor consisting of a mixture of roderite phase and of prespinel phase, said precursor resulting from the mixing of two ternary precursors each comprising copper, aluminum and zinc, one of said ternary precursors containing at least 65% by weight of roderite phase and the other one of said ternary precursors containing at least 65% by weight of prespinel phase.

19. A process according to one of claims 1 to 17 for preparing a precursor of catalyst containing copper, aluminum and zinc, at least 85% by weight of said precursor consisting of a mixture of roderite phase and of prespinel phase, said precursor resulting from the mixing of two ternary precursors each comprising copper, aluminum and zinc, one of said ternary precursors containing at least 85% by weight of roderite phase and the other one of said ternary precursors containing at least 85% by weight of prespinel phase.

20. A process according to one of claims 1 to 19 for preparing a hydroxycarbonated precursor of catalyst wherein said mixing is achieved between two hydroxycarbonated ternary precursors.

21. A process for preparing a catalyst, characterized in that it comprises the following stages:

1) grinding of the precursor prepared according to one of claims 1 to 20 in order to obtain a product in the form of a powder with a grain size smaller than 1 mm,
2) shaping of the product obtained at the end of stage 1), optionally including mixing said product at a rate of 0.5-5% by weight with at least one compound selected from the group consisting of graphite, stearic acid, the stearates,
3) calcination of the product obtained at the end of stage 2), between 250 and 500°C.

22. A precursor containing 15 to 80% by weight of copper, 4 to 50% aluminum and 10 to 70% zinc in relation to total weight of the metals present in said precursor, a part of these elements being in roderite phase, characterized in that the other part of these elements is in prespinel phase having an atomic ratio (Cu + Zn)/Al of between 0.20 and 2.10 and an atomic ratio Zn/Al of between 0.10 and 1.50 and showing, in infra-red spectrum, a $(CO_3^{2-})$ vibration leading to a wide band, the maximum of which is located at 1500 cm$^{-1}$ and a narrow band at 1390 cm$^{-1}$, said phases representing more than 50% by weight of the precursor.

23. A catalyst containing 15 to 80% by weight copper, 4 to 50% aluminum and 10 to 70% zinc, in relation to total weight of metals present in said catalyst, a part of these elements being in roderite phase, characterized in that the other part of those elements is in prespinel phase, having an atomic ratio (Cu + Zn)/Al of between 0.20 and 2.10 and an atomic ratio Zn/Al of between 0.10 and 1.50 and showing, in infra-red spectrum, a $(CO_3^{2-})$ vibration leading to a wide band, the maximum of which is located at 1500 cm$^{-1}$ and a narrow band at 1390 cm$^{-1}$.

24. Use of a catalyst according to claim 23 or prepared according to claim 21 in a process utilizing a balanced reaction of the carbon oxides CO and $CO_2$ with hydrogen.

**25.** Use of a catalyst according to claim 23 or prepared according to claim 21 in a process for producing methanol from carbon oxides CO and $CO_2$ and hydrogen.

**26.** Use of a catalyst according to claim 23, or prepared according to claim 21 in a process for decomposing at least one $C_1$ to $C_3$ primary alcohol into carbone oxides CO and $CO_2$ and hydrogen.

## Patentansprüche

**1.** Verfahren zur Herstellung eines Katalysator-Vorläufers, der Kupfer, Aluminium und Zink enthält, wobei mindestens 50 Gew.-% des Vorläufers bestehen aus einem Gemisch aus einer Roderit genannten Phase, bestehend aus Kupfer, Aluminium und Zink und mit einem Atomverhältnis (Cu+Zn)/Al zwischen 3,5 und 16 und einem Atomverhältnis Zn/Al zwischen 1,6 und 6, und einer mikrokristallisierter Prespinell genannten Phase, bestehend aus Kupfer, Aluminium und Zink und mit einem Atomverhältnis (Cu+Zn)/Al zwischen 0,20 und 2,10 und einem Atomverhältnis Zn/Al zwischen 0,10 und 1,50, dadurch gekennzeichnet, daß der Vorläufer resultiert aus dem Mischen von zwei ternären Vorläufern, die jeweils Kupfer, Aluminium und Zink enthalten, wobei einer dieser ternären Vorläufer mindestens 50 Gew.-% Roderit-Phase enthält und der andere dieser ternären Vorläufer mindestens 50 Gew.-% mikrokristallisierte Prespinell-Phase enthält, wobei miteinander gemischt werden
einerseits einer der ternären Vorläufer, wie sie am Ende einer der Stufen (1) bis (4) eines die folgenden Stufen umfassenden Verfahrens P erhalten werden:

(1) Durchführung einer Copräzipitation im Innern eines Reaktors, der vorher Wasser enthält, zwischen einer wäßrigen Lösung mindestens einer Verbindung eines Vertreters, ausgewählt aus der Gruppe, die besteht aus den Alkalimetallen und dem Ammoniumion, wobei die Verbindung ausgewählt wird aus der Gruppe, die besteht aus den Carbonaten, den Hydrogencarbonaten und den Hydroxiden, und einer wäßrigen Lösung, die in Form von Verbindungen, ausgewählt aus der Gruppe, die besteht aus den löslichen Salzen und den löslichen Komplexen, Kupfer, Aluminium und Zink in solchen Mengenverhältnissen enthält, daß das Atomverhältnis (Cu + Zn)/Al zwischen 3,5 und 16 liegt und das Atomverhältnis Zn/Al zwischen 1,6 und 6 liegt, wobei die Reaktion bei einem pH-Wert zwischen 6,3 und 7,3 und bei einer Temperatur zwischen 70 und 90°C mit einer Verweilzeit zwischen 0,1 und 60 min stattfindet,
(2) Filtrieren und anschließendes Waschen mit Wasser des am Ende der Stufe (1) erhaltenen Produkts,
(3) Trocknen des am Ende der Stufe (2) erhaltenen Produkts zwischen 50 und 200°C, und
(4) Calcinieren des am Ende der Stufe (3) erhaltenen Produkts zwischen 250 und 500°C, und

andererseits einer der ternären Vorläufer, wie sie am Ende einer der Stufen (1) bis (4) eines die folgenden Stufen umfassenden Verfahrens P' erhalten werden: (1) Durchführung einer Copräzipitation im Innern eines Reaktors, der vorher Wasser enthält, zwischen einer wäßrigen Lösung mindestens einer Verbindung eines Vertreters, ausgewählt aus der Gruppe, die besteht aus den Alkalimetallen und dem Ammoniumion, wobei die Verbindung ausgewählt wird aus der Gruppe, die besteht aus den Carbonaten, den Hydrogencarbonaten und den Hydroxiden, und einer wäßrigen Lösung, die in Form von Verbindungen, ausgewählt aus der Gruppe, die besteht aus den löslichen Salzen und den löslichen Komplexen, Kupfer, Aluminium und Zink in solchen Mengenverhältnissen enthält, daß das Atomverhältnis (Cu + Zn)/Al zwischen 0,20 und 2,10 und das Atomverhältnis Zn/Al zwischen 0,10 und 1,50 liegt, wobei die Reaktion bei einem pH-Wert zwischen 6,3 und 7,3 und bei einer Temperatur zwischen 45 und 65°C mit einer Verweilzeit zwischen 0,1 und 60 min stattfindet, (2) Filtrieren und dann Waschen mit Wasser des am Ende der Stufe (1) erhaltenen Produkts, (3) Trocknen des am Ende der Stufe (2) erhaltenen Produkts zwischen 50 und 200°C, und (4) Calcinieren des am Ende der Stufe (3) erhaltenen Produkts zwischen 250 und 500°C,
wobei auf das Mischen folgen:

- ein Filtrieren und ein Waschen mit Wasser, dann ein Trocknen zwischen 50 und 200°C, dann ein Calcinieren zwischen 250 und 500°C, wenn mindestens einer der beiden ternären Vorläufer, die miteinander gemischt worden sind, vorher am Ende der Stufe (1) nach einem der Verfahren P und P' erhalten worden ist;

- ein Trocknen zwischen 50 und 200°C und dann ein Calcinieren zwischen 250 und 500°C, wenn einer der beiden ternären Vorläufer, die miteinander gemischt worden sind, vorher am Ende der Stufe (2) eines der Verfahren P und P' erhalten worden ist und der andere der beiden ternären Vorläufer, die miteinander gemischt worden sind, am Ende einer der Stufen (2) bis (4) eines der Verfahren P und P' erhalten worden ist; und

- ein Calcinieren zwischen 250 und 500°C, wenn die beiden ternären Vorläufer, die miteinander gemischt worden sind, vorher am Ende einer der Stufen (3) und (4) eines der Verfahren P und P' erhalten worden sind.

**2.** Verfahren nach Anspruch 1, dadurch gekennzeichnet, daß es die folgenden Stufen umfaßt:

a) Durchführung einer Copräzipitation gemäß Stufe (1) des Verfahrens P des Anspruchs 1,
b) Filtrieren und dann Waschen mit Wasser des am Ende der Stufe (a) erhaltenen Produkts,
c) Durchführung einer Copräzipitation gemäß Stufe (1) des Verfahrens P' des Anspruchs 1,
d) Filtrieren und dann Waschen mit Wasser des am Ende der Stufe (c) erhaltenen Produkts,
e) Mischen des am Ende der Stufe (b) erhaltenen Produkts mit dem am Ende der Stufe (d) erhaltenen Produkt,
f) Trocknen des am Ende der Stufe (e) erhaltenen Produkts zwischen 50 und 200°C und
g) Calcinieren des am Ende der Stufe (f) erhaltenen Produkts zwischen 250 und 500°C.

**3.** Verfahren nach Anspruch 1, dadurch gekennzeichnet, daß es die folgenden Stufen umfaßt:

a) Durchführung einer Copräzipitation gemäß Stufe (1) des Verfahrens P des Anspruchs 1,
b) Filtrieren und dann Waschen mit Wasser des am Ende der Stufe (a) erhaltenen Produkts,
c) Trocknen des am Ende der Stufe (b) erhaltenen Produkts zwischen 50 und 200°C,
d) Durchführung einer Copräzipitation gemäß Stufe (1) des Verfahrens P' des Anspruchs 1,
e) Filtrieren und dann Waschen mit Wasser des am Ende der Stufe (d) erhaltenen Produkts,
f) Trocknen des am Ende der Stufe (e) erhaltenen Produkts zwischen 50 und 200°C und
g) Mischen des am Ende der Stufe (c) erhaltenen Produkts mit dem am Ende der Stufe (f) erhaltenen Produkt und
h) Calcinieren des am Ende der Stufe (g) erhaltenen Produkts zwischen 250 und 500°C.

**4.** Verfahren nach Anspruch 1, dadurch gekennzeichnet, daß es die folgenden Stufen umfaßt:

a) Durchführung einer Copräzipitation gemäß Stufe (1) des Verfahrens P nach Anspruch 1,
b) Filtrieren und dann Waschen mit Wasser des am Ende der Stufe (a) erhaltenen Produkts,
c) Trocknen des am Ende der Stufe (b) erhaltenen Produkts zwischen 50 und 200°C,
d) Calcinieren des am Ende der Stufe (c) erhaltenen Produkts zwischen 250 und 500°C,
e) Durchführung einer Copräzipitation gemäß Stufe (1) des Verfahrens P' des Anspruchs 1,
f) Filtrieren und dann Waschen mit Wasser des am Ende der Stufe (e) erhaltenen Produkts,
g) Trocknen des am Ende der Stufe (f) erhaltenen Produkts zwischen 50 und 200°C,
h) Calcinieren des am Ende der Stufe (g) erhaltenen Produkts zwischen 250 und 500°C,
i) Mischen des am Ende der Stufe (d) erhaltenen Produkts mit dem am Ende der Stufe (h) erhaltenen Produkt und
j) Calcinieren des am Ende der Stufe (i) erhaltenen Produkts zwischen 250 und 500°C.

**5.** Verfahren nach Anspruch 1, dadurch gekennzeichnet, daß es die folgenden Stufen umfaßt:

a) Durchführung einer Copräzipitation gemäß Stufe (1) des Verfahrens P des Anspruchs 1,
b) Filtrieren und dann Waschen mit Wasser des am Ende der Stufe (a) erhaltenen Produkts,
c) Trocknen des am Ende der Stufe (b) erhaltenen Produkts zwischen 50 und 200°C,
d) Durchführung einer Copräzipitation gemäß Stufe (1) des Verfahrens P' des Anspruchs 1,
e) Filtrieren und dann Waschen mit Wasser des am Ende der Stufe (d) erhaltenen Produkts,
f) Mischen des am Ende der Stufe (c) erhaltenen Produkts mit dem am Ende der Stufe (e) erhaltenen Produkt,
g) Trocknen des am Ende der Stufe (h) erhaltenen Produkts zwischen 50 und 200°C und
h) Calcinieren des am Ende der Stufe (g) erhaltenen Produkts zwischen 250 und 500°C.

**6.** Verfahren nach Anspruch 1, dadurch gekennzeichnet, daß es die folgenden Stufen umfaßt:

a) Durchführung einer Copräzipitation gemäß Stufe (1) des Verfahrens P des Anspruchs 1,
b) Filtrieren und dann Waschen mit Wasser des am Ende der Stufe (a) erhaltenen Produkts,
c) Trocknen des am Ende der Stufe (b) erhaltenen Produkts zwischen 50 und 200°C,
d) Calcinieren des am Ende der Stufe (c) erhaltenen Produkts zwischen 250 und 500°C,
e) Durchführung einer Copräzipitation gemäß Stufe (1) des Verfahrens P' des Anspruchs 1,
f) Filtrieren und dann Waschen mit Wasser des am Ende der Stufe (e) erhaltenen Produkts,
g) Mischen des am Ende der Stufe (d) erhaltenen Produkts mit dem am Ende der Stufe (f) erhaltenen Produkt,
h) Trocknen des am Ende der Stufe (g) erhaltenen Produkts zwischen 50 und 200°C und
i) Calcinieren des am Ende der Stufe (h) erhaltenen Produkts zwischen 250 und 500°C.

7. Verfahren nach Anspruch 1, dadurch gekennzeichnet, daß es die folgenden Stufen umfaßt:

    a) Durchführung einer Copräzipitation gemäß Stufe (1) des Verfahrens P' des Anspruchs 1,
    b) Filtrieren und dann Waschen mit Wasser des am Ende der Stufe (a) erhaltenen Produkts,
    c) Trocknen des am Ende der Stufe (b) erhaltenen Produkts zwischen 50 und 200°C,
    d) Durchführung einer Copräzipitation gemäß Stufe (1) des Verfahrens P des Anspruchs 1,
    e) Filtrieren und dann Waschen mit Wasser des am Ende der Stufe (d) erhaltenen Produkts,
    f) Mischen des am Ende der Stufe (c) erhaltenen Produkts mit dem am Ende der Stufe (e) erhaltenen Produkt,
    g) Trocknen des am Ende der Stufe (h) erhaltenen Produkts zwischen 50 und 200°C und
    h) Calcinieren des am Ende der Stufe (g) erhaltenen Produkts zwischen 250 und 500°C.

8. Verfahren nach Anspruch 1, dadurch gekennzeichnet, daß es die folgenden Stufen umfaßt:

    a) Durchführung einer Copräzipitation gemäß Stufe (1) des Verfahrens P' des Anspruchs 1,
    b) Filtrieren und dann Waschen mit Wasser des am Ende der Stufe (a) erhaltenen Produkts,
    c) Trocknen des am Ende der Stufe (b) erhaltenen Produkts zwischen 50 und 200°C,
    d) Calcinieren des am Ende der Stufe (c) erhaltenen Produkts zwischen 250 und 500°C,
    e) Durchführung einer Copräzipitation gemäß Stufe (1) des Verfahrens P des Anspruchs 1,
    f) Filtrieren und dann Waschen mit Wasser des am Ende der Stufe (e) erhaltenen Produkts,
    g) Mischen des am Ende der Stufe (d) erhaltenen Produkts mit dem am Ende der Stufe (f) erhaltenen Produkt,
    h) Trocknen des am Ende der Stufe (g) erhaltenen Produkts zwischen 50 und 200°C und
    i) Calcinieren des am Ende der Stufe (h) erhaltenen Produkts zwischen 250 und 500°C.

9. Verfahren nach Anspruch 1, dadurch gekennzeichnet, daß es die folgenden Stufen umfaßt:

    a) Durchführung einer Copräzipitation gemäß Stufe (1) des Verfahrens P des Anspruchs 1,
    b) Filtrieren und dann Waschen mit Wasser des am Ende der Stufe (a) erhaltenen Produkts,
    c) Trocknen des am Ende der Stufe (b) erhaltenen Produkts zwischen 50 und 200°C,
    d) Calcinieren des am Ende der Stufe (c) erhaltenen Produkts zwischen 250 und 500°C,
    e) Durchführung einer Copräzipitation gemäß Stufe (1) des Verfahrens P' des Anspruchs 1,
    f) Filtrieren und dann Waschen mit Wasser des am Ende der Stufe (e) erhaltenen Produkts,
    g) Trocknen des am Ende der Stufe (f) erhaltenen Produkts zwischen 50 und 200°C,
    h) Mischen des am Ende der Stufe (d) erhaltenen Produkts mit dem am Ende der Stufe (g) erhaltenen Produkt und
    i) Calcinieren des am Ende der Stufe (h) erhaltenen Produkts zwischen 250 und 500°C.

10. Verfahren nach Anspruch 1, dadurch gekennzeichnet, daß es die folgenden Stufen umfaßt:

    a) Durchführung einer Copräzipitation gemäß Stufe (1) des Verfahrens P' des Anspruchs 1,
    b) Filtrieren und dann Waschen mit Wasser des am Ende der Stufe (a) erhaltenen Produkts,
    c) Trocknen des am Ende der Stufe (b) erhaltenen Produkts zwischen 50 und 200°C,
    d) Calcinieren des am Ende der Stufe (c) erhaltenen Produkts zwischen 250 und 500°C,
    e) Durchführung einer Copräzipitation gemäß Stufe (1) des Verfahrens P des Anspruchs 1,
    f) Filtrieren und dann Waschen mit Wasser des am Ende der Stufe (e) erhaltenen Produkts,
    g) Trocknen des am Ende der Stufe (g) erhaltenen Produkts zwischen 50 und 200°C,
    h) Mischen des am Ende der Stufe (d) erhaltenen Produkts mit dem am Ende der Stufe (g) erhaltenen Produkt und
    i) Calcinieren des am Ende der Stufe (h) erhaltenen Produkts zwischen 250 und 500°C.

11. Verfahren nach Anspruch 1, dadurch gekennzeichnet, daß es die folgenden Stufen umfaßt:

    a) Durchführung einer Copräzipitation gemäß Stufe (1) des Verfahrens P des Anspruchs 1,
    b) Durchführung einer Copräzipitation gemäß Stufe (1) des Verfahrens P' des Anspruchs 1,
    c) Mischen des am Ende der Stufe (a) erhaltenen Produkts mit dem am Ende der Stufe (b) erhaltenen Produkt,
    d) Filtrieren und dann Waschen mit Wasser des am Ende der Stufe (c) erhaltenen Produkts,
    e) Trocknen des am Ende der Stufe (d) erhaltenen Produkts zwischen 50 und 200°C und
    f) Calcinieren des am Ende der Stufe (e) erhaltenen Produkts zwischen 250 und 500°C.

12. Verfahren zur Herstellung eines Katalysatorvorläufers nach einem der Ansprüche 1 bis 11, dadurch gekennzeichnet, daß miteinander gemischt werden
einerseits der am Ende der Stufe (2) des Verfahrens P erhaltene Vorläufer, der in Wasser suspendiert worden ist, und
andererseits der am Ende der Stufe (2) des Verfahrens P' erhaltene ternäre Vorläufer,
wobei die Stufe (1) des Verfahrens P' in der Suspension des ternären Vorläufers des Verfahrens P durchgeführt wird und das erhaltene Gemisch filtriert und dann mit Wasser gewaschen, zwischen 50 und 200°C getrocknet und dann zwischen 250 und 500°C calciniert wird.

13. Verfahren zur Herstellung eines Katalysatorvorläufers nach einem der Ansprüche 1 bis 11, dadurch gekennzeichnet, daß man miteinander mischt
einerseits den am Ende der Stufe (2) des Verfahrens P' erhaltenen ternären Vorläufer, der in Wasser suspendiert worden ist, und
andererseits den am Ende der Stufe (2) des Verfahrens P erhaltenen ternären Vorläufer,
wobei die Stufe (1) des Verfahrens P in der Suspension des ternären Vorläufers des Verfahrens P' durchgeführt wird, das erhaltene Gemisch filtriert und dann mit Wasser gewaschen, zwischen 50 und 200°C getrocknet und dann zwischen 250 und 500°C calciniert wird.

14. Verfahren nach einem der Ansprüche 1, 6 und 8 bis 10, das zwei Calcinierungsstufen umfaßt, wobei auf die erste Calcinierungsstufe unmittelbar folgt ein Zerkleinern (Mahlen) des am Ende der ersten Calcinierungsstufe erhaltenen Produkts zur Herstellung eines Produkts in Form eines Pulvers mit einer Korngrößenverteilung von kleiner als $2 \times 10^{-6}$ m

15. Verfahren nach einem der Ansprüche 1 und 4, das drei Calcinierungsstufen umfaßt, bei dem auf mindestens eine der beiden ersten Calcinierungsstufen unmittelbar folgt ein Zerkleinern (Mahlen) des am Ende einer der beiden ersten Calcinierungsstufen erhaltenen Produkts zur Herstellung eines Produkts in Form eines Pulvers mit einer Korngrößenverteilung von kleiner als $2 \times 10^{-6}$ m.

16. Verfahren nach einem der Ansprüche 1 bis 15, bei dem auf mindestens eine Trocknungsstufe unmittelbar folgt ein Zerkleinern (Mahlen) des am Ende dieser Trocknungsstufe erhaltenen Produkts zur Herstellung eines Produkts in Form eines Pulvers mit einer Korngrößenverteilung von kleiner als $2 \times 10^{-6}$ m.

17. Verfahren nach einem der Ansprüche 1 bis 15, bei dem auf jede Trocknungsstufe unmittelbar folgt ein Zerkleinern (Mahlen) des am Ende der Trocknungsstufe erhaltenen Produkts zur Herstellung eines Produkts in Form eines Pulvers mit einer Korngrößenverteilung von kleiner als $2 \times 10^{-6}$ m.

18. Verfahren nach einem der Ansprüche 1 bis 17 zur Herstellung eines Katalysatorvorläufers, der Kupfer, Aluminium und Zink enthält, wobei mindestens 65 Gew.-% des Vorläufers bestehen aus einem Gemisch aus einer Roderit-Phase und einer Prespinell-Phase, wobei der aus dem Mischen der beiden ternären Vorläufer resultierende Vorläufer jeweils Kupfer, Aluminium und Zink enthält, wobei einer der ternären Vorläufer mindestens 65 Gew.-% Roderit-Phase und der andere der ternären Vorläufer mindestens 65 Gew.-% Prespinell-Phase enthält.

19. Verfahren nach einem der Ansprüche 1 bis 17 zur Herstellung eines Katalysatorvorläufers, der Kupfer, Aluminium und Zink enthält, wobei mindestens 85 Gew.-% des Vorläufers bestehen aus einem Gemisch aus einer Roderit-Phase und einer Prespinell-Phase, wobei der aus dem Mischen der beiden ternären Vorläufer resultierende Vorläufer jeweils Kupfer, Aluminium und Zink enthält, wobei einer der ternären Vorläufer mindestens 85 Gew.-% Roderit-Phase und der andere der ternären Vorläufer mindestens 85 Gew.-% Prespinellphase enthält.

20. Verfahren nach einem der Ansprüche 1 bis 19 zur Herstellung eines Hydroxycarbonat-haltigen Katalysatorvorläufers, bei dem zwei Hydroxycarbonat-haltige ternäre Vorläufer miteinander gemischt werden.

21. Verfahren zur Herstellung eines Katalysators, dadurch gekennzeichnet, daß es die folgenden Stufen umfaßt:

(1) Zerkleinern (Mahlen) des nach einem der Ansprüche 1 bis 20 hergestellten Vorläufers zur Herstellung eines Produkts in Form eines Pulvers mit einer Korngrößenverteilung von kleiner als 1 mm;

(2) Formen des am Ende der Stufe (1) erhaltenen Produkts, das gegebenenfalls umfaßt eine Mischung des Produkts in einer Menge von 0,5 bis 5 % seines Gewichts mit mindestens einer Verbindung, ausgewählt aus der Gruppe, die besteht aus Graphit, Stearinsäure und Stearaten, und

(3) Calcinieren des am Ende der Stufe (2) erhaltenen Produkts zwischen 250 und 500°C.

22. Vorläufer, enthaltend 15 bis 80 Gew.-% Kupfer, 4 bis 50 Gew.-% Aluminium und 10 bis 70 Gew.-% Zink, bezogen auf das Gesamtgewicht der in dem Vorläufer vorhandenen Metalle, wobei ein Teil dieser Elemente in einer Roderit-Phase vorliegt, dadurch gekennzeichnet, daß der andere Teil dieser Elemente in einer Prespinell-Phase die ein Atomverhältnis $(Cu + Zn)/Al$ zwischen 0,20 und 2,10 und ein Atomverhältnis $Zn/Al$ zwischen 0,10 und 1,50 aufweist und im Infrarotspektrum eine Schwingung $(CO_3^{2-})$ aufweist, die zu einer breiten Bande, deren Maximum bei 1500 $cm^{-1}$ liegt, und zu einer schmalen Bande bei 1390 $cm^{-1}$ führt, vorliegt, wobei diese Phasen mehr als 50 % des Gewichtes des Vorläufers ausmachen.

23. Katalysator, enthaltend 15 bis 80 Gew.-% Kupfer, 4 bis 50 Gew.-% Aluminium und 10 bis 70 Gew.-% Zink, bezogen auf das Gesamtgewicht der in dem Katalysator vorliegenden Metalle, wobei ein Teil dieser Elemente in Form einer Roderit-Phase vorliegt, dadurch gekennzeichnet, daß der andere Teil dieser Elemente in Form einer Prespinell-Phase, die ein Atomverhältnis $(Cu + Zn)/Al$ zwischen 0,20 und 2,10 und ein Atomverhältnis $Zn/Al$ zwischen 0,10 und 1,50 aufweist und im Infrarotspektrum eine Schwingung $(CO_3^{2-})$ aufweist, die zu einer breiten Bande, deren Maximum bei 1500 $cm^{-1}$ liegt, und zu einer schmalen Bande bei 1390 $cm^{-1}$ führt, vorliegt.

24. Verwendung eines Katalysators nach Anspruch 23 oder eines nach Anspruch 21 hergestellten Katalysators in einem Verfahren, bei dem eine Gleichgewichtsreaktion zwischen den Kohlenstoffoxiden CO und $CO_2$ und Wasserstoff angewendet wird.

25. Verwendung eines Katalysators nach Anspruch 23 oder eines nach Anspruch 21 hergestellten Katalysators in einem Verfahren zur Herstellung von Methanol aus den Kohlenstoffoxiden CO und $CO_2$ und Wasserstoff.

26. Verwendung eines Katalysators nach Anspruch 23 oder eines nach Anspruch 21 hergestellten Katalysators in einem Verfahren zur Zersetzung mindestens eines primären Alkohols mit 1 bis 5 Kohlenstoffatomen zu den Kohlenstoffoxiden CO und $CO_2$ und Wasserstoff.

FIG.1

FIG.2

FIG.3

## FIG.4

## FIG.5